# EUROPEAN PATENT APPLICATION

(11) **EP 4 821 491 A1**
(43) Date of publication of application: **14.10.2026**
(21) Application number: 26181160.8
(22) Date of filing: 22.10.2021
(51) Int. Cl.: A61K 9/51, A61K 47/54, A61K 47/69

(54) **HYDROPHOBIC DRUGS IN ORGANIC CORE HIGH DENSITY LIPOPROTEIN (HDL) NANOPARTICLES**

(30) Priority: 23.10.2020 US 202063105206 P
(62) Document number(s) of the earlier application(s) in accordance with Art. 76 EPC: 21884013.0 / 4 232 092
(71) Applicant: Northwestern University, Evanston, IL 60208-0001 (US); City of Hope, Duarte, CA 91010 (US)
(72) Inventor: HENRICH, Stephen E., Chicago, IL 60657 (US); RINK, Jonathan S., Park Ridge, IL 60068 (US); LIN, Adam Y., Evenston, IL 60208 (US); THAXTON, C. Shad, Chicago, IL 60614 (US); GORDON, Leo I., Winnetka, IL 60093 (US); NGUYEN, Sonbinh T., Evanston, IL 60201 (US); ROSEN, Steven T., Duarte, CA 91010 (US); HORNE, David, Duarte, CA 91010 (US); ZHANG, Xu Hannah, Duarte, CA 91010 (US)
(74) Representative: D Young & Co LLP

(57) **Abstract**

Disclosed herein are high-density lipoprotein-like nanoparticles (HDL-NP) having a soft material core (*e.g.,* a lipid-conjugated inorganic core) associated with hydrophobic therapeutic agents. In some embodiments, the HDL-NPs are targeted to scavenger receptor type B1 (SR-B1). In some embodiments, the hydrophobic therapeutic agents are chemotherapeutic agents. Also disclosed herein are methods for treating disorders such as cancer with the HDL-NPs.

## Description

### STATEMENT REGARDING FEDERALLY SPONSORED

### RESEARCH OR DEVELOPMENT

This invention was made with government support under grant number CA233922 awarded by the National Institutes of Health. The government has certain rights in the invention.

### RELATED APPLICATION

This application claims priority under 35 U.S.C. § 119(e) to United States Provisional
Application Serial Number 63/105,206, filed October 23, 2020, the entire contents of which
are incorporated by reference herein.

### BACKGROUND OF THE INVENTION

Standard treatment regimens for cancer typically include chemotherapeutic agents to reduce tumor burden. Often, these therapeutics are hydrophobic, resulting in problems with formulation, biodistribution, bioavailability, pharmacokinetics and delivery of a sufficient drug load to reduce tumor burden. For example, the hydrophobic drug F7 (also referred to as PIK-75) inhibits the p100 subunit of PI3K as well as DNA-PK, and can induce cell death in malignant cells. However, given its hydrophobic nature, the therapeutic application compounds with its profile (*e.g.*, hydrophobicity) are limited. Previous efforts in the field have attempted to encapsulate hydrophobic drugs in the context of lipid vesicles, such as liposomes; however, while this approach has shown reduced side-effects the reduction was at the expense of reduced efficacy as the encapsulated drugs remain untargeted to the tumor tissue. Further, using present methods, drug loading is restricted to the bilayer membrane of the liposome vesicles and there is no availability for drug loading in the core of these types of materials.

### SUMMARY OF THE INVENTION

The present disclosure utilizes synthetic lipoprotein-like biologics (HDL-NPs) that have the potential to mimic high-density lipoproteins and target tumor cells through scavenger receptor type B1 (SR-B1), based on their size, shape, surface composition, and charge. In addition, the core of these materials can be tuned to accommodate hydrophobic drugs. To synthesize these nanoparticles, an organic core (PL₄) is employed as a scaffold to assemble the drug (*e.g.*, F7), which is formulated with phospholipids, and a protein called apolipoprotein (*e.g.*, apolipoprotein A-I (ApoA-I)) which assists in targeting the nanoparticles. These nanoparticle constructs are stable, capable of delivering drugs in complex matrices such as serum containing media, and efficiently induce cancer cell death.

Additionally, the present disclosure provides methods for the synthesis and characterization of an HDL mimic using lipid-conjugated organic core scaffolds. The core design motif constrains and orients phospholipid geometry to facilitate the assembly of soft-core nanoparticles that in some embodiments are approximately 10 nm in diameter and resemble human HDLs in their size, shape, surface chemistry, composition and protein secondary structure.

Accordingly, in some aspects, the disclosure relates to a high-density lipoprotein nanoparticle (HDL-NP) comprising: (a) an organic core (core); (b) a shell surrounding and attached to the core wherein the core comprises a hydrophobic phospholipid conjugated scaffold (PL₄); and (c) a hydrophobic therapeutic agent associated with one or more of the organic core or shell.

In some embodiments, the HDL-NP further comprises an apolipoprotein. In some embodiments, the apolipoprotein is apolipoprotein A-I, apolipoprotein A-II, or apolipoprotein E. In some embodiments, the apolipoprotein is apolipoprotein A-I (Apo-I).

In some embodiments, the hydrophobic therapeutic agent is associated to the organic core, shell, or apolipoprotein non-covalently or through hydrophobic interactions.

In some embodiments, the shell is attached to the organic core non-covalently. In some embodiments, the shell is attached to the organic core through hydrophobic interactions.

In some embodiments, the PL₄ comprises a headgroup-modified phospholipid. In some embodiments, the headgroup-modified phospholipid comprises a ring-strained alkyne, 1,2-dipalmitoyl-*sn*-glycero-3-phosphoethan-olamine-N-dibenzocyclooctyl.

In some embodiments, the organic core scaffold comprises an amphiphilic DNA-linked small molecule-phospholipid conjugate (DNA- PL₄).

In some embodiments, the HDL-NP has a diameter of about 5-30 nm, 5-20 nm, 5-15 nm, 5-10 nm, 8-13 nm, 8-12 nm, or 10 nm.

In some embodiments, the HDL-NP has a zeta potential closer to human HDL than a synthetic HDL nanoparticle with a gold core.

In some embodiments, the HDL-NP has a hydrodynamic diameter of 8.7 nm-17-7nm. In some embodiments, the HDL-NP has a hydrodynamic diameter of 12 nm-14nm.

In some embodiments, the hydrophobicity of a hydrophobic therapeutic agent is measured (*e.g.*, quantified, assessed) by a partitioning method to establish a partition coefficient (P). In some embodiments, the hydrophobic therapeutic agent has a partition coefficient (P) of greater than or equal to 0 (*e.g.*, log P ≥0). In some embodiments, the hydrophobic therapeutic agent has a partition coefficient (P) of greater than or equal to 0.25 (*e.g.*, log P ≥0.25). In some embodiments, the hydrophobic therapeutic agent has a partition coefficient (P) of greater than or equal to 0.5 (*e.g.*, log P ≥0.5). In some embodiments, the hydrophobic therapeutic agent has a partition coefficient (P) of greater than or equal to 1 (*e.g.*, log P ≥1). In some embodiments, the hydrophobic therapeutic agent has a partition coefficient (P) of greater than or equal to 2 (*e.g.*, log P ≥2).

In some embodiments, the hydrophobic therapeutic agent is an anti-cancer agent. In some embodiments, the hydrophobic therapeutic agent is a chemotherapeutic agent. In some embodiments, the HDL-NP comprises an additional therapeutic agent. In some embodiments, the hydrophobic therapeutic agent comprises PIK75 (F7)
(C₁₆H₁₄BrN₅O₄S.HCl), doxorubicin, vincristine, gemcitabine, paclitaxel, docetaxel, andrographolide, sutent, tamoxifen, or a combination thereof. In some embodiments, the hydrophobic therapeutic agent is PIK75 (F7) (C₁₆H₁₄BrN₅O₄S.HCl). In some embodiments, the hydrophobic therapeutic agent has a structure of Formula (I) (CAS No. 372196-77-5).

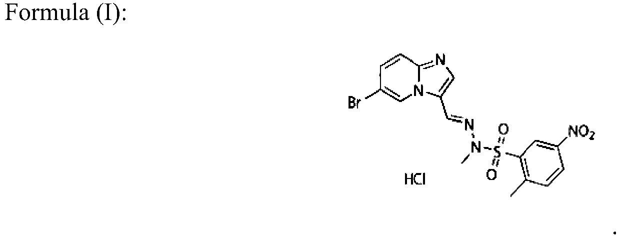

In some aspects, the disclosure relates to a pharmaceutical composition comprising any of the HDL-NPs as described herein.

In some aspects, the disclosure relates to a method of delivering a hydrophobic therapeutic agent to a cell (*e.g.,* a cancer cell) comprising surface receptor scavenger receptor type B1 (SR-B1) in a subject, the method comprising administering to a subject an effective amount of at least one of any one of the HDL-NPs and/or the compositions of the present disclosure.

In some aspects, the disclosure relates to a method for treating a cancer, the method comprising administering to a subject having a cancer at least one of any one of the HDL-NPs and/or the compositions of the present disclosure in an effective amount to treat the cancer.

In some embodiments, the subject of any of the methods as described herein is a mammal. In some embodiments, the subject of any of the methods as described herein is a human.

In some embodiments, the subject of any of the methods as described herein has cancer. In some embodiments, the subject of any of the methods as described herein has one or more of renal cancer, chronic myeloid leukemia (CML), multiple myeloma (MM), adult acute myeloid leukemia (AML), acute lymphocytic leukemia (ALL), cutaneous T cell lymphoma (CTCL), melanoma, ovarian cancer, breast cancer, gastrointestinal malignancies, brain tumors, prostate cancer, or colon cancer. In some embodiments, the subject of any of the methods as described herein has cutaneous T cell lymphoma. In some embodiments, the subject of any of the methods as described herein has renal cancer. In some embodiments, the subject of any of the methods as described herein has colon cancer. In some embodiments, the subject of any of the methods as described herein has prostate cancer. In some embodiments, the subject has renal cancer.

In some embodiments, the effective amount of the hydrophobic therapeutic agent necessary to treat the subject having cancer is decreased relative to a control. In some embodiments, the effective amount of the hydrophobic therapeutic agent necessary to treat the subject having cancer is decreased by at least 5%, 10%, 25%, 40%, 50%, 75%, or more, relative to a control. In some embodiments, a control is a control subject. In some embodiments, a control subject is being treated with the same hydrophobic therapeutic agent delivered in the absence of an HDL-NP.

In some embodiments, the HDL-NPs and/or the composition cause reduced cytotoxicity of non-cancerous cells in the subject and/or reduced symptoms, relative to a control. In some embodiments, a control is a control subject. In some embodiments, a control subject is being treated with a standard-of-care or alternative treatment.

These and other aspects and embodiments will be described in greater detail herein. The description of some exemplary embodiments of the disclosure are provided for illustration purposes only and not meant to be limiting. Additional compositions and methods are also embraced by this disclosure.

The summary above is meant to illustrate, in a non-limiting manner, some of the embodiments, advantages, features, and uses of the technology disclosed herein. Other embodiments, advantages, features, and uses of the technology disclosed herein will be apparent from the Detailed Description, Drawings, Examples, and Claims.

### BRIEF DESCRIPTION OF THE DRAWINGS

The following drawings form part of the present specification and are included to further demonstrate certain aspects of the present disclosure, which can be better understood by reference to one or more of these drawings in combination with the detailed description of specific embodiments presented herein. For purposes of clarity, not every component may be labeled in every drawing. It is to be understood that the data illustrated in the drawings in no way limit the scope of the disclosure. In the drawings:

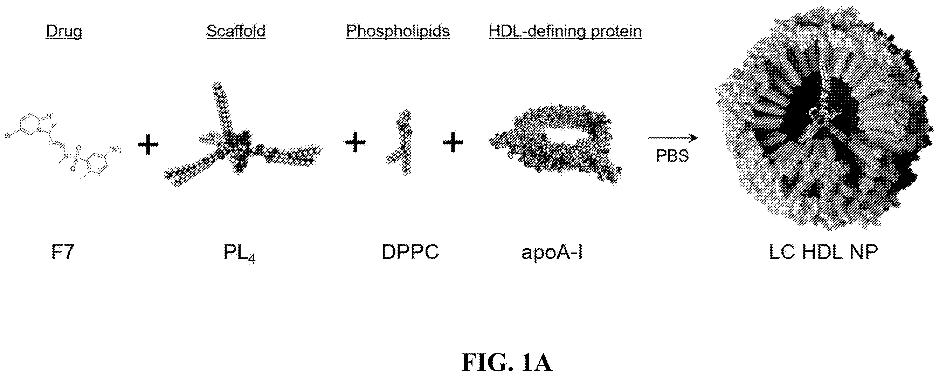

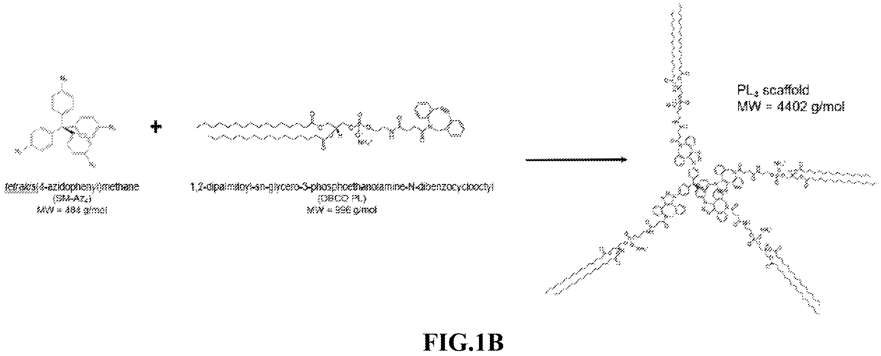

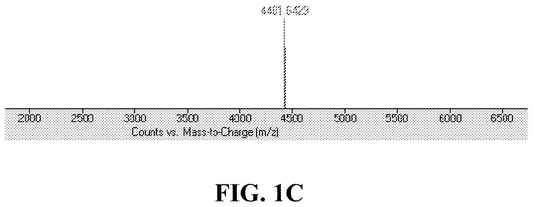

**FIGs. 1A-1C** show an assembly schematic for an HDL-NP carrying a hydrophobic therapeutic agent, an assemble schematic for PL₄ and properties thereof. FIG 1A shows an assembly schematic for an HDL-NP carrying a hydrophobic therapeutic agent. First a scaffold (PL₄) and hydrophobic drug (*e.g.*, hydrophobic therapeutic agent) thin film is prepared. Then a phosphatidylcholine (PC) liposome is prepared as thin film formation. The liposomes are added to the core scaffold at a molar ratio of 20:1. The apolipoprotein A-I (Apo-AI) is added at a 2:1 molar ratio to core scaffold and is sonicated (90 seconds on, 30 seconds off, by three times. The resultant is rested for 30 minutes on ice and then concentrated by filtration using a 50 kDa MWCO spin column. FIG. 1B shows a PL₄ synthesis scheme. PL₄ core materials were synthesized by copper-free click chemistry conjugation of 1,2-dipalmitoyl-sn-glycero-3-phosphoethanolamine-N-dibenzocyclooctyl (DBCO PE) with a tetrahedral small molecule core (tetrakis(4-azidophenyl)methane) with four terminal azides. In a typical reaction, the DBCO PE and tetrakis(4-azidophenyl)methane were each dissolved at 0.1 wt % in N,N-dimethylformamide (DMF, Sigma Aldrich) and mixed at a 10:1 molar ratio of DBCO PE to *tetrakis*(4-azidophenyl)methane in DMF. The reaction mixture was subjected to three rounds of alternating vortexing and bath sonication and was then allowed to react at room temperature under vortex for 24 hours. HPLC and electrospray ionization mass spectrometry (FIG. 1C) was then used to characterize the resulting reaction mixture. FIG. 1C shows an electrospray ionization mass spectrometry of a PL₄ core. Product m/z = 4401.6 (Theoretical: 4401.7). As FIG. 1C only shows a single species at the right mass for PL₄, we conclude that there is no partially coupled product (PL₃, PL₂, etc.) and use the reaction mixture for the assembly step. As the assembly step involves adding a large excess of DPPC lipids, there is no need to separate the excess DBCO PE molecule from the PL₄ core prior to its use in the assembly.

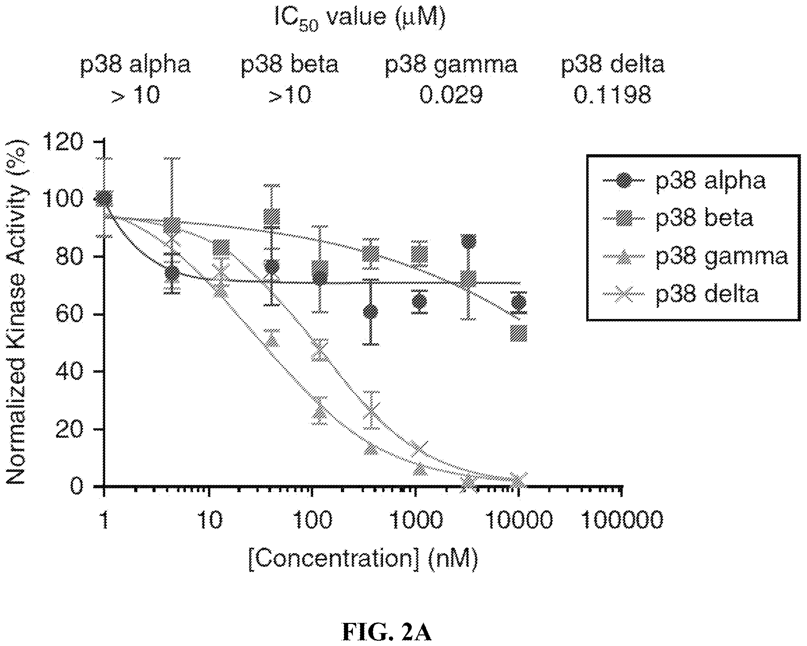

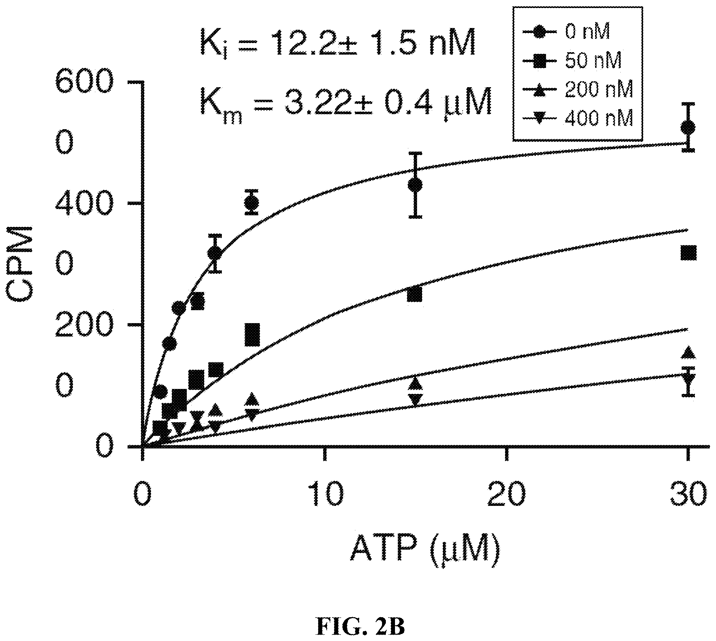

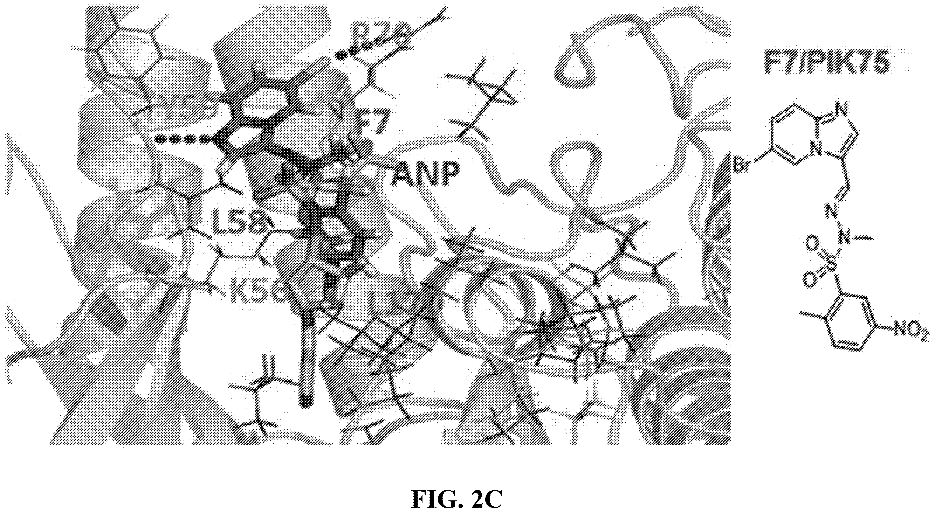

**FIGs. 2A-2C** show that PIK75 (*e.g.*, F7) targets p38gamma (p38γ) kinase activity *in vitro* and in an ATP-dependent manner. FIG. 2A: An ADP-Glo *in vitro* kinase assay was used to calculate IC₅₀ for PIK75 inhibition of kinas activity of the four p38 isoforms, normalized to DMSO control. Calculated IC₅₀, values (micromole/Liter) are indicated. FIG. 2B: Time-resolved fluorescence energy transfer was used to measure *in vitro* enzyme kinetics of inhibition of p38γ kinase at indicated concentrations of PIK75. Counts per minute (CPM) corresponds to product formation level. The error bar of the measurements represents standard deviation of triplicate data. Solid lines represent data fitting to the competitive inhibition model. Calculated Kᵢ and Kₘ are indicated. FIG. 2C: Mapping of the CSPs induced by PIK75 binding to p38γ on the docked structure of p38γ in complex with PIK75. PIK75 forms three hydrogen bonds with K56/Y59/R70, which are displayed as blue dots. The ANP molecule x-ray structure is displayed as grey sticks for comparison. The residues with the largest line-broadening effects are indicated in red, and those with significant CSPs (> 0.05 ppm) are indicated in green with their sidechains shown in stick form. L58 and L170 are within a 3-angstrom distance of PIK75. ANP, an analogue of ATP molecule with extra nitrogen bonds (a non-hydrolyzable ATP analogue); ATP, adenosine triphosphate; SCP, chemical shift perturbation; Ctrl, control; IC50, half maximal inhibitory concentration; Kᵢ, enzyme constants for inhibitors; Kₘ, enzyme constants for substrates; M, mol/L; SS, Sezary syndrome.

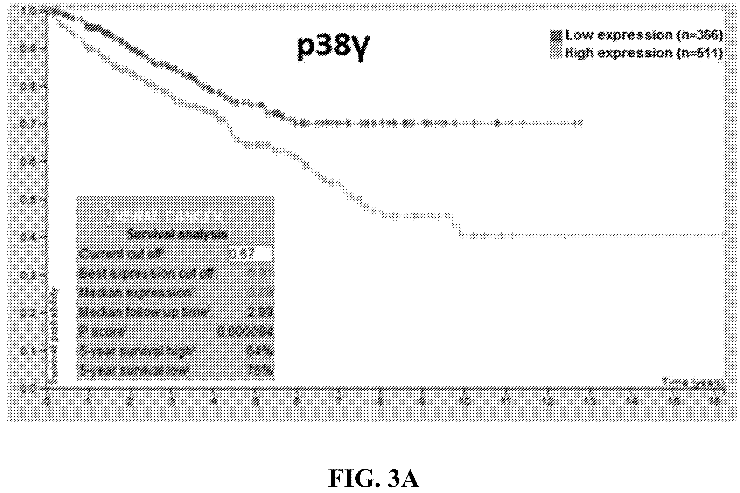

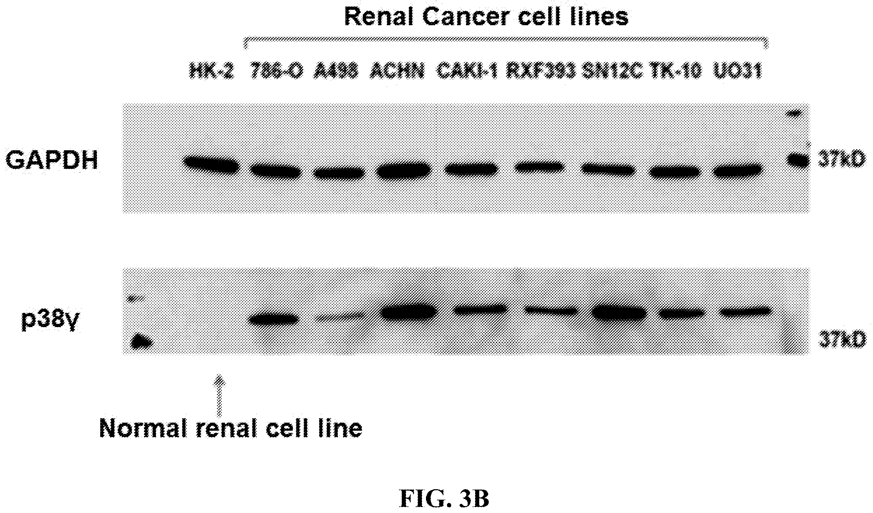

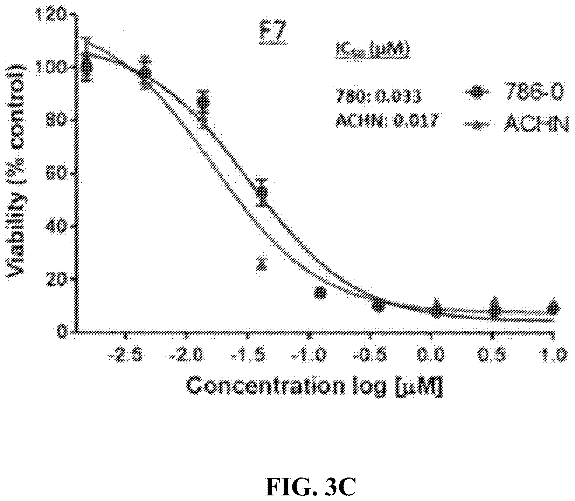

**FIGs. 3A-3C** shows that PIK75 (F7) targets renal cancer cell lines. FIG. 3A: shows a survival curve for renal cancer is very well separated in (P=0.000084) summarized 877 renal cancer patients, patients who had low expression of p38gamma (p38γ) (n=366) exhibited longer life spans than that of high expression of p38gamma (p38γ). FIG. 3B: shows that all 8 renal cancer cell lines have a higher p38gamma (p38γ) protein expression level than that of normal HK2 renal cells by western blot. FIG. 3C: shows 2 renal cancer cell lines, 780 and ACHN, that are sensitive to PIK75 with cytotoxicity IC₅₀ of 33nM and 17nM, respectively.

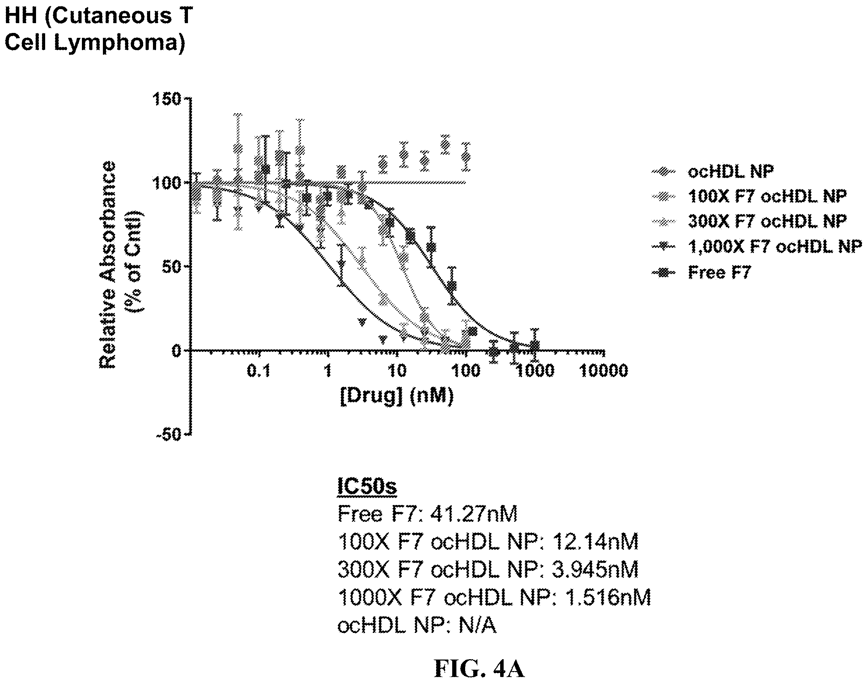

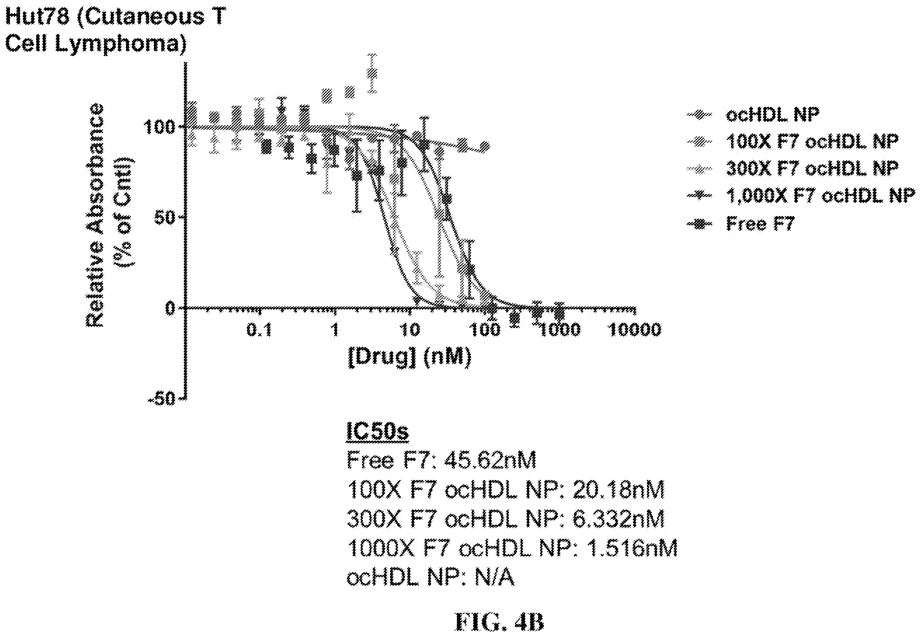

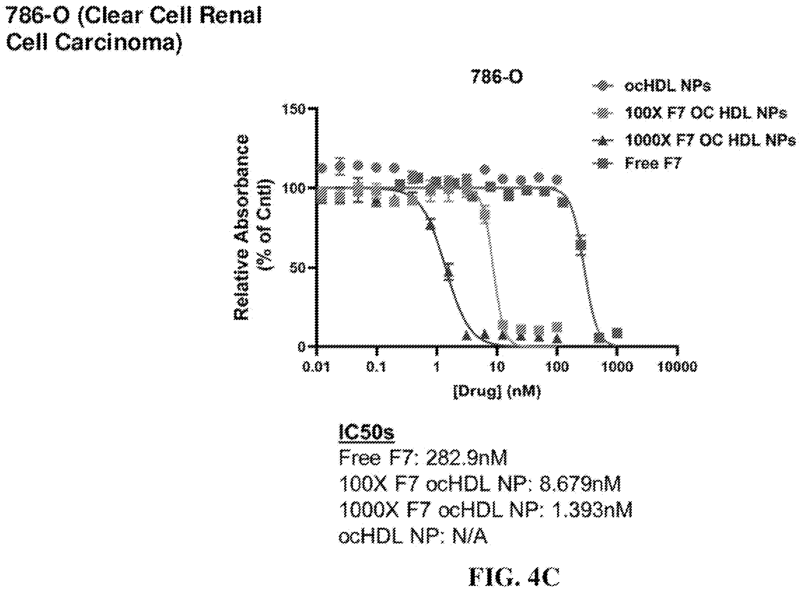

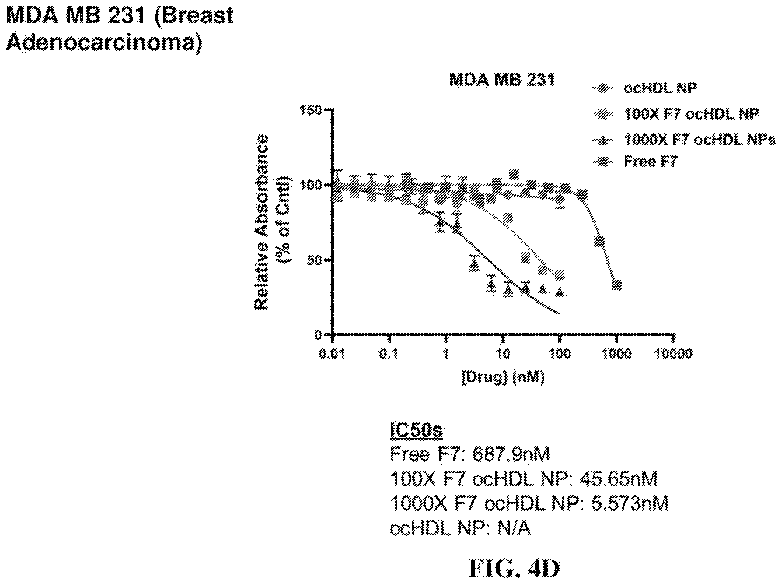

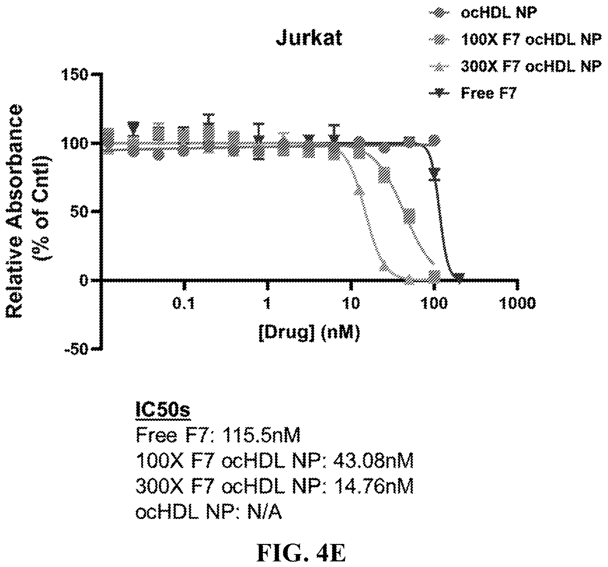

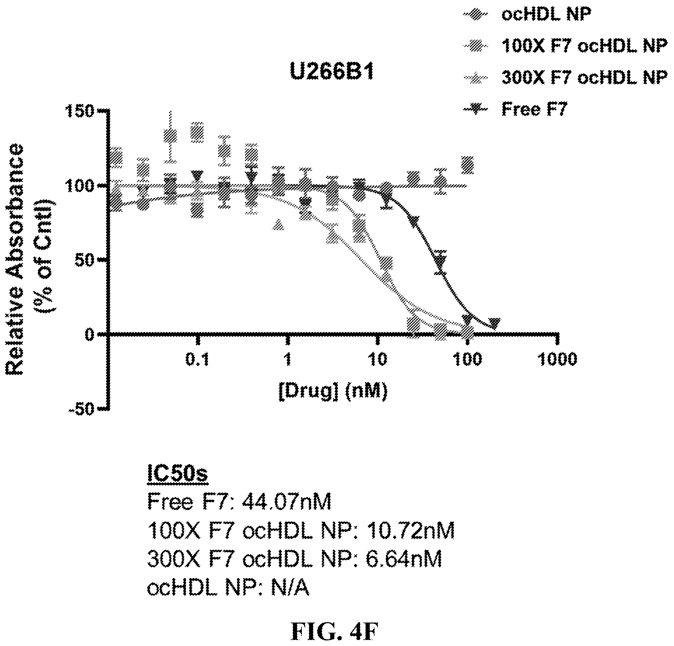

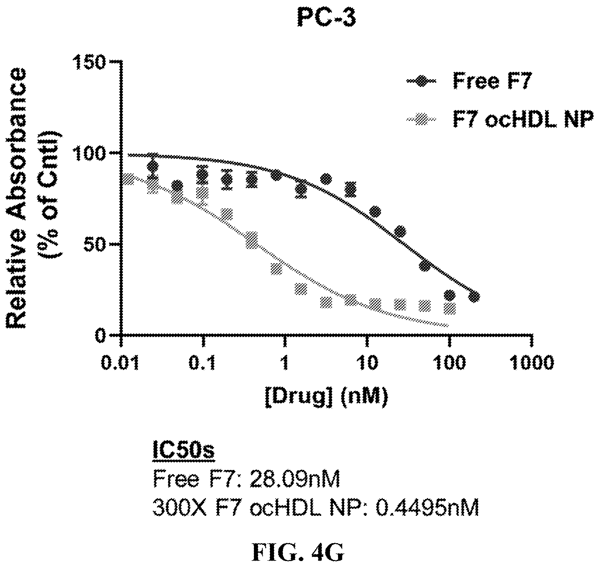

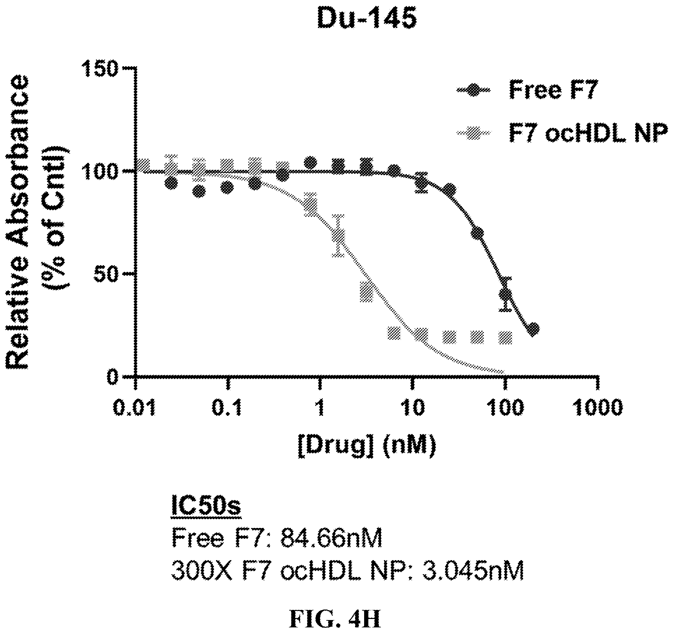

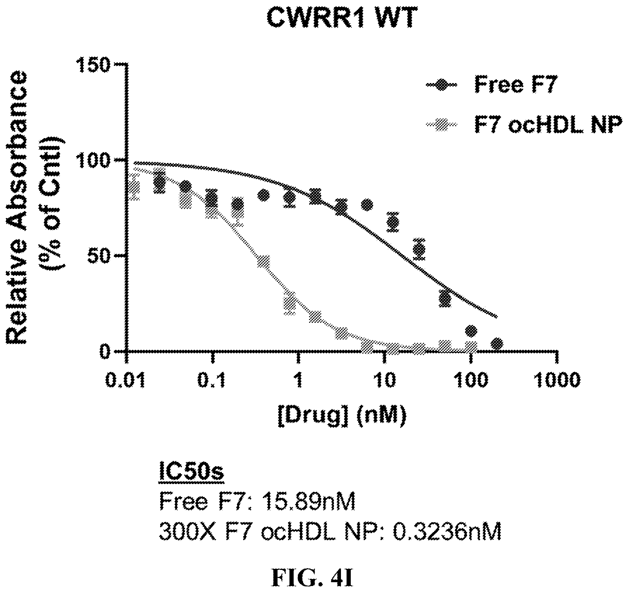

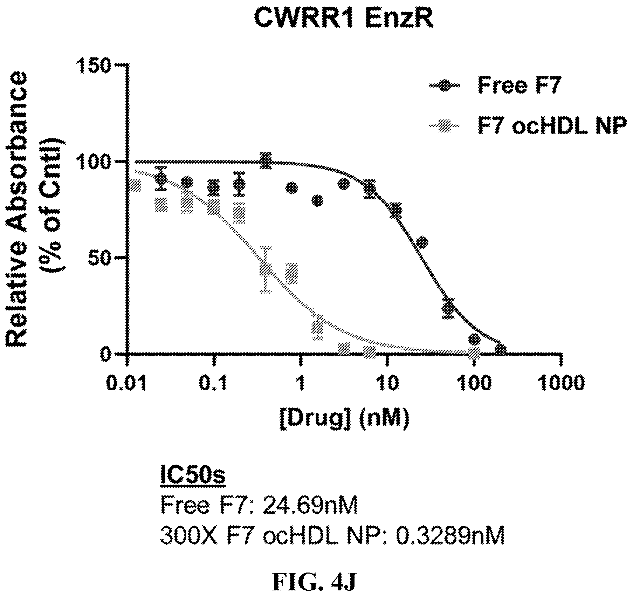

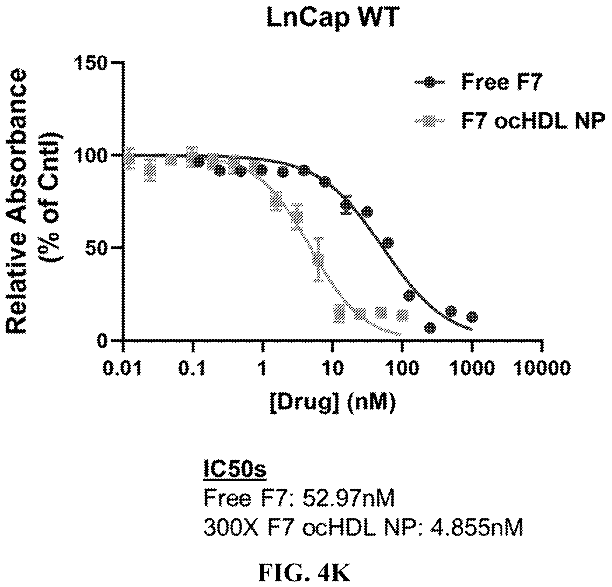

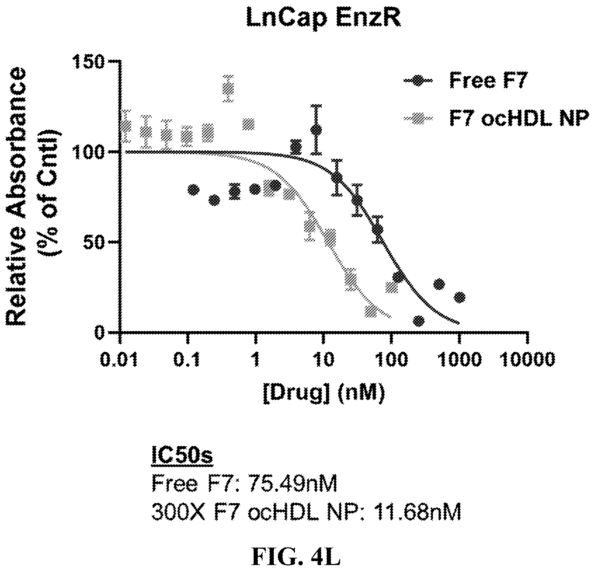

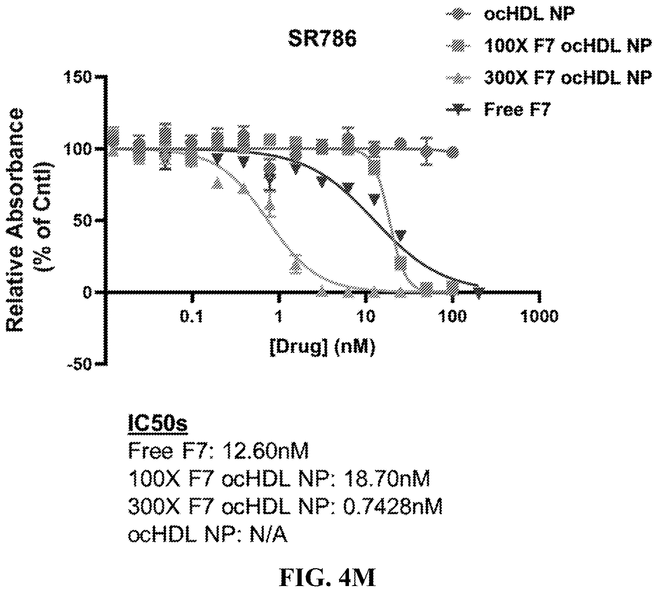

**FIGs. 4A-4D** shows that the HDL-NPs encapsulating the hydrophobic therapeutic agents (*e.g.*, PIK75) exhibit and retain similar cytotoxicity IC₅₀ as that of original PIK75 (free) toward multiple cancer cell lines. FIG. 4A shows the efficacy of PIK75 loaded HDL-NPs in HH cells (cutaneous T cell lymphoma). FIG. 4B shows the efficacy of PIK75 loaded HDL-NPs in Hut78 cells (cutaneous T cell lymphoma). FIG. 4C shows the efficacy of PIK75 loaded HDL-NPs in 786-O cells (clear cell renal cell carcinoma). FIG. 4D shows the efficacy of PIK75 loaded HDL-NPs in MDA MB 231 cells (breast cancer). FIG. 4E shows the efficacy of PIK75 loaded HDL-NPs in Jurkat cells (T cell lymphoma). FIG. 4F shows the efficacy of PIK75 loaded HDL-NPs in U266B1 cells (myeloma). FIG. 4G shows the efficacy of PIK75 loaded HDL-NPs in PC-3 cells (prostate cancer). FIG. 4H shows the efficacy of PIK75 loaded HDL-NPs in Du-145 cells (prostate cancer). FIG. 4I shows the efficacy of PIK75 loaded HDL-NPs in CWRR1 WT cells (prostate cancer). FIG. 4J shows the efficacy of PIK75 loaded HDL-NPs in CRWW1 EnzR cells (prostate cancer). FIG. 4K shows the efficacy of PIK75 loaded HDL-NPs in LnCap WT cells (prostate cancer). FIG. 4L shows the efficacy of PIK75 loaded HDL-NPs in LnCap EnzR cells (prostate cancer). FIG. 4M shows the efficacy of PIK75 loaded HDL-NPs in SR-786 cells (anaplastic large T cell lymphoma).

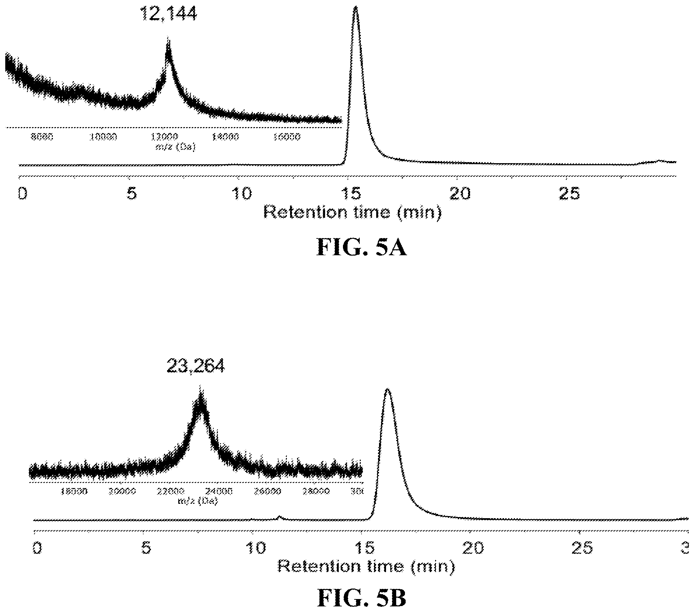

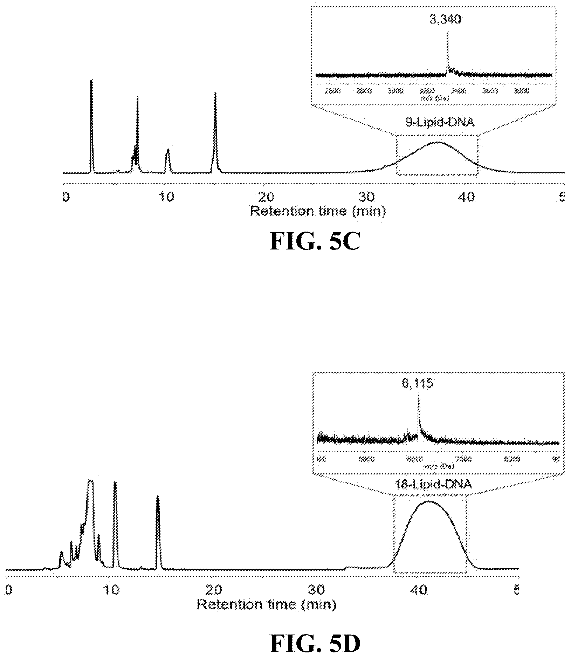

**FIGs. 5A-5D** shows analyses of 9-SMDH₄, 18-SMDH_{4,} crude 9-DNA-lipid, and crude 18-DNA-lipid. FIG. 5A: an analytical RP-HPLC trace of 9-SMDH₄ (SEQ ID NO: 1) from the coupling reaction of the *tetrakis*(4-azidophenyl) methane with alkyne-functionalized 9-mer DNAs on the CPGs. The trace is the signal from the diode detector set at 260 nm. Inset shows the MALDI-ToF spectrum of the pure product: m/z =12,144 (12,144.1 theoretical). FIG. 5B: shows an analytical RP-HPLC trace of 18-SMDH₄ (SEQ ID NO: 2) from the coupling reaction of the *tetrakis*(4-azidophenyl) methane with alkyne-functionalized 18-mer DNAs on the CPGs. The trace is the signal from the diode detector set at 260 nm. Inset shows the MALDI-ToF spectrum of the pure product: m/z = 23,264 (23,263.7 theoretical). FIG. 5C: shows a semi-preparative RP-HPLC trace of crude 9-DNA-lipid (SEQ ID NO: 3). The trace is the signal from the diode detector set at 260 nm. The pure 9-DNA-lipid (SEQ ID NO: 3) at 33-42 min was isolated and identified by MALDI-ToF (Inset): m/z = 3,340 (3,342.4 theoretical). FIG. 5D: shows a semi-preparative RP-HPLC trace of crude 18-DNA-lipid (SEQ ID NO: 4). The trace is the signal from the diode detector set at 260 nm. The pure 18-DNA-lipid (SEQ ID NO: 4) at 38-45 min was isolated and identified by MALDI-ToF (Inset): m/z = 6,115 (6,118.2 theoretical).

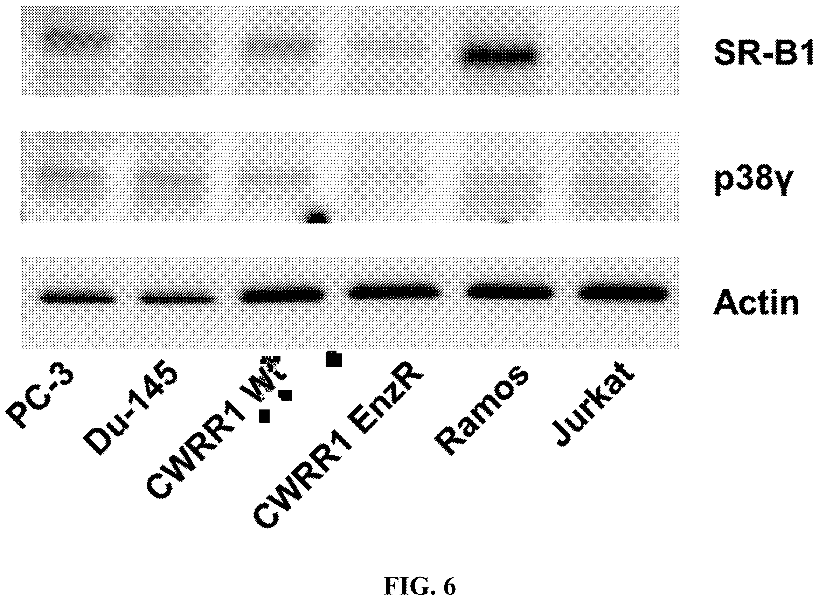

**FIG. 6** shows the expression levels of SR-B1 and p38γ in several prostate cancer cell lines using a western blot analysis.

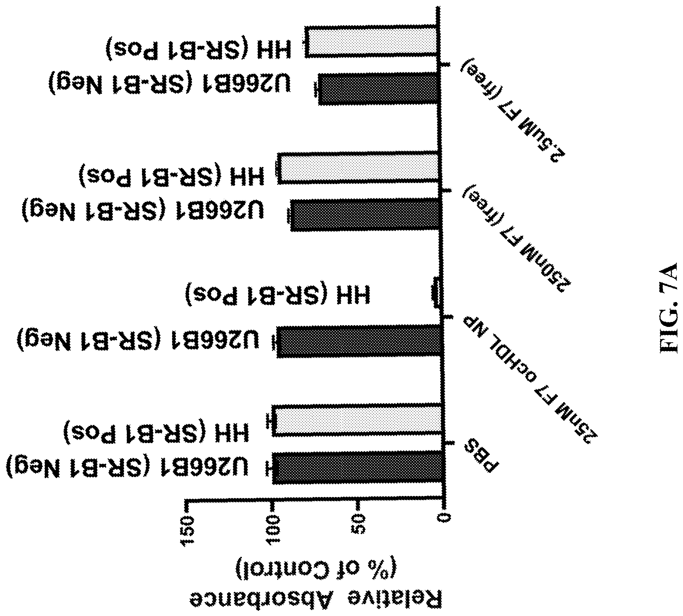

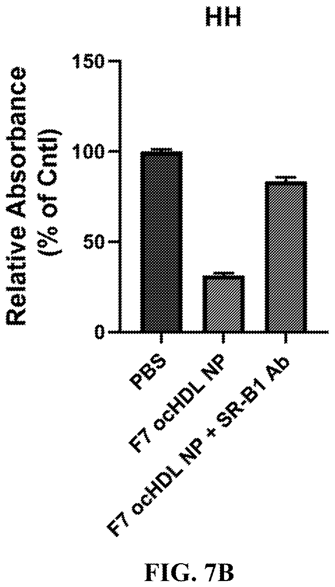

**FIGs. 7A-7B** show that delivery of HDL NPs loaded with F7 can occur via receptor (SR-B1) mediated uptake. FIG. 7A shows cytotoxicity data of SR-B1 positive cells (HH cell line) and SR-B1 negative cells (U266B1 cell line) following treatment with HDL NPs loaded with F7. FIG. 7B shows cytotoxicity data of SR-B1 positive cells (HH cell line) following treatment with HDL NPs loaded with F7 in the presence or absence of a SR-B1 blocking antibody.

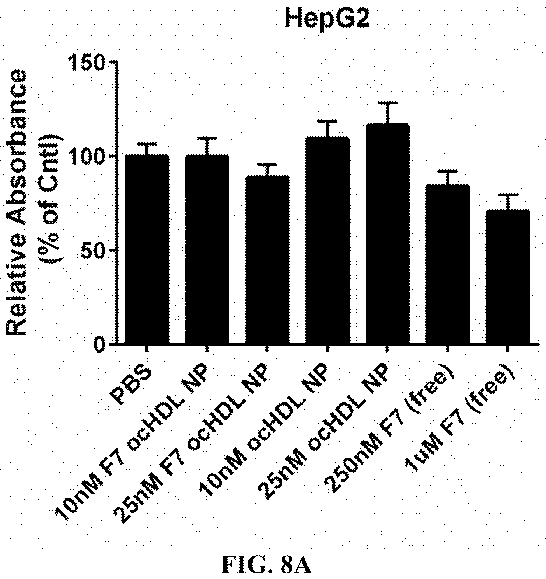

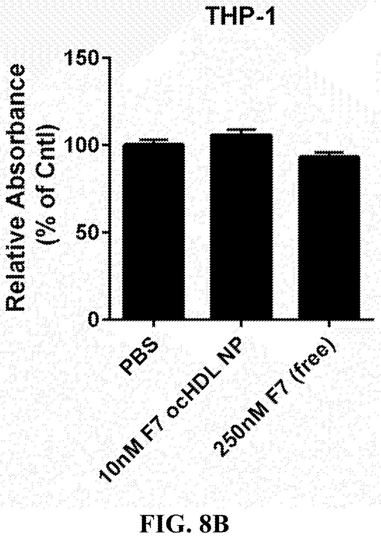

**FIG. 8A-8B** show the results of a cell-based toxicity study of HDL NPs loaded with F7. FIG. 8A shows cytotoxicity data of HepG2 cells following treatment with HDL NPs loaded with F7. FIG. 8B shows cytotoxicity data of THP-1 cells (SR-B1 positive) following treatment with HDL NPs loaded with F7.

### DETAILED DESCRIPTION OF THE INVENTION

Previously, other methods have used liposomal encapsulation to improve the pharmacokinetics of hydrophobic drugs. These formulations have ultimately been shown to have limited upside, as they don't materially alter patient outcomes. Additionally, liposomes are only passively - not actively - targeted to malignant cells. High-density lipoproteins (HDL) are native circulating nanoparticles that carry cholesterol, target specific cell types, and play important roles in a host of disease processes. As a result, synthetic HDL mimics have become promising therapeutic agents. Native HDLs are circulating nanoparticles (~8-13 nm in diameter) that transport cholesterol and play important roles in cancer and cardiovascular disease.

Herein, it was unexpectedly found that the hydrophobic therapeutic agents (*e.g.*, PIK75) could be encapsulated in HDL-like nanoparticles having lipid-conjugated organic core scaffolds. These HDL-like nanoparticles of the present disclosure are the first of their kind, namely HDL mimics with hydrophobic therapeutic agents encapsulated therein, which have a similar cytotoxicity profile when evaluated against their free (unbound) counterparts. The HDL-NPs of the instant disclosure, can interact with various lipoprotein receptors typically overexpressed in cancer (*e.g.*, scavenger receptor type B1), which can aid in targeting the nanoparticles and the drug to malignant cells. Furthermore, the lipid-conjugated organic core scaffolds can be configured (*e.g.*, constructed, modified) to have different hydrophobicity values. For example, without limitation, nucleic acids (*e.g.*, DNA) may be integrated into the core to increase the hydrophobicity in the core. As such, herein successful particle fabrication is shown using three different organic core scaffolds. Specifically, a tetrahedral small molecule-phospholipid hybrid, called PL₄, and a tetrahedral ssDNA-phospholipid-small molecule hybrid, called DNA-PL₄ using two different length nucleic acids.

The HDL-like nanoparticles of the present disclosure mimic HDL species using lipid-conjugated organic core scaffolds. The core design motif constrains and orients phospholipid geometry to facilitate the assembly of soft-core nanoparticles that are, in some embodiments, approximately 10 nm in diameter and resemble human HDLs in their size, shape, surface chemistry, composition and protein secondary structure. The HDL-like nanoparticles mimic the structure of native HDL with respect to size (~10 nm), surface chemistry (-20 mV zeta potential), and HDL protein secondary structure as determined by circular dichroism. Synthetic HDL-NPs have demonstrated promise as therapy for cardiovascular disease and cancer, among other indications.

Herein, the synthesis of HDL mimetic nanoparticles using lipid-conjugated core scaffolds (HDL NPs) is accomplished in a two-step process: first, the core scaffolds are synthesized and purified; second, the particle is fabricated via supramolecular assembly of the core scaffold, free phospholipids, and the HDL-defining protein, apolipoprotein A1 (apo-A1). A variety of lipid-conjugated organic cores can theoretically be used for particle assembly. Herein, successful particle fabrication using a tetrahedral small molecule-phospholipid hybrid, called PL₄ is used.

The present disclosure provides methods for the synthesis of HDL-like nanoparticles with structural and functional properties of mature human HDLs using lipid-conjugated core scaffolds (HDL NP). An organic scaffold using a highly hydrophobic small molecule-phospholipid conjugate (PL₄) was synthesized using copper-free click chemistry. Specifically, a headgroup-modified phospholipid harboring a ring-strained alkyne, 1,2-dipalmitoyl-*sn*-glycero-3-phosphoethan-olamine-N-dibenzocyclooctyl, was click coupled to *tetrakis*(4-az-idophenyl)methane, a small molecule with four terminal azides (SM-Az4) (FIGs. 1B-1C). As used herein, the terms "HDL-like", "HDL-mimetic", and "HDL mimic" are used interchangeably to refer to a synthetic HDL-NP of the disclosure.

In some aspects, the disclosure relates to a high-density lipoprotein nanoparticle (HDL-NP) comprising: (a) an organic core (core); (b) a shell surrounding and attached to the core wherein the core comprises a hydrophobic phospholipid conjugated scaffold (PL₄); and (c) a hydrophobic therapeutic agent associated with one or more of the organic core or shell.

In some embodiments, the HDL-NP further comprises an apolipoprotein. In some embodiments, the apolipoprotein is apolipoprotein A-I, apolipoprotein A-II, or apolipoprotein E. In some embodiments, the apolipoprotein is apolipoprotein A-I (Apo-I).

The shell may be formed, at least in part, of one or more components, such as a plurality of lipids, which may optionally associate with one another and/or with surface of the organic core. For example, components (*e.g.*, shell, lipid shell) may be associated with the organic core by being covalently or non-covalently attached to the organic core, physiosorbed, chemisorbed, or attached to the organic core through ionic interactions, hydrophobic and/or hydrophilic interactions, electrostatic interactions, van der Waals interactions, or combinations thereof. In some embodiments, the shell is non-covalently attached to the organic core. In some embodiments, the shell is attached to the organic core by hydrophobic interactions.

In some embodiments, the hydrophobic therapeutic agent is associated (*e.g.*, by any of the means described herein) with the organic core. In some embodiments, the hydrophobic therapeutic agent is associated (*e.g.*, by any of the means described herein) with the shell. In some embodiments, the hydrophobic therapeutic agent is associated (*e.g.*, by any of the means described herein) to the organic core and the shell. In some embodiments, as described elsewhere herein, the HDL-NP comprises an apolipoprotein, in some such embodiments, the hydrophobic therapeutic agent is associated with an apolipoprotein. In some embodiments, the hydrophobic therapeutic agent is associated to the organic core and an apolipoprotein. In some embodiments, the hydrophobic therapeutic agent is associated to the organic a shell and an apolipoprotein. In some embodiments, the hydrophobic therapeutic agent is associated to an organic core, shell, and an apolipoprotein. In some embodiments, as described elsewhere herein, the HDL-NP comprises additional components, in some such embodiments, the hydrophobic therapeutic agent is associated with any additional component. In some embodiments, the hydrophobic therapeutic agent is associated to the outer layer of a shell. In some embodiments, the hydrophobic therapeutic agent is associated to the inner layer of a shell. In some embodiments, the attachment is by hydrophobic interactions. In some embodiments, the attachment is a non-covalent attachment.

A nanoparticle may comprise any number of therapeutic agents as can be attached to the nanoparticle. In some embodiments, a nanoparticle comprises at least 5 units or molecules of therapeutic agents per core (*e.g.,* a ratio of 5:1 agents:core, *e.g.,* 5:1 agents:PLA₄ core). In some embodiments, a nanoparticle comprises at least 10, at least 20, at least 40, at least 50, at least 75, at least 100, at least 150, at least 200, at least 250, at least 300, at least 400, at least 500, at least 600, at least 700, at least 800, at least 900, at least 1,000, at least 1,100, at least 1,200, at least 1,300, at least 1,400, at least 1,500, or at least 2,000 units or molecules of therapeutic agents per core (*e.g.,* a ratio of 1000:1 agents:core, *e.g.,* 1000:1 F7:PLA₄ core). In some embodiments, a nanoparticle comprises a ratio of at least 5:1, at least 10:1, at least 20:1, at least 40:1, at least 50:1, at least 75:1, at least 100:1, at least 150:1, at least 200:1, at least 250:1, at least 300:1, at least 400:1, at least 500:1, at least 600:1, at least 700:1, at least 800:1, at least 900:1, at least 1,000:1, at least 1,100:1, at least 1,200:1, at least 1,300:1, at least 1,400:1, at least 1,500:1, or at least 2,000:1 units or molecules of therapeutic agents relative to core. In some embodiments, a nanoparticle comprises a ratio of at least 5:1, at least 10:1, at least 20:1, at least 40:1, at least 50:1, at least 75:1, at least 100:1, at least 150:1, at least 200:1, at least 250:1, at least 300:1, at least 400:1, at least 500:1, at least 600:1, at least 700:1, at least 800:1, at least 900:1, at least 1,000:1, at least 1,100:1, at least 1,200:1, at least 1,300:1, at least 1,400:1, at least 1,500:1, or at least 2,000:1 units or molecules of therapeutic agents relative to PL₄ core.

One or more lipids and/or lipid analogues may form a single layer or a multi-layer (*e.g.*, a bilayer) of a structure. In some instances where multi-layers are formed, the natural or synthetic lipids or lipid analogs interdigitate (*e.g.*, between different layers). Non-limiting examples of natural or synthetic lipids or lipid analogs include fatty acyls, glycerolipids, glycerophospholipids, sphingolipids, saccharolipids and polyketides (derived from condensation of ketoacyl subunits), and sterol lipids and prenol lipids (derived from condensation of isoprene subunits).

In one particular set of embodiments, a structure described herein includes one or more phospholipids. The one or more phospholipids may include, for example, phosphatidylcholine, phosphatidylglycerol, lecithin, β, γ-dipalmitoyl-α-lecithin, sphingomyelin, phosphatidylserine, phosphatidic acid, N-(2,3-di(9-(Z)-octadecenyloxy))-prop-1-yl-N,N,N-trimethylammonium chloride, phosphatidylethanolamine, lysolecithin, lysophosphatidylethanolamine, phosphatidylinositol, cephalin, cardiolipin, cerebrosides, dicetylphosphate, dioleoylphosphatidylcholine, dipalmitoylphosphatidylcholine, dipalmitoylphosphatidylglycerol, dioleoylphosphatidylglycerol, palmitoyl-oleoyl-phosphatidylcholine, di-stearoyl-phosphatidylcholine, stearoyl-palmitoyl-phosphatidylcholine, di-palmitoyl-phosphatidylethanolamine, di-stearoyl-phosphatidylethanolamine, di-myrstoyl-phosphatidylserine, di-oleyl-phosphatidylcholine, 1,2-dipalmitoyl-sn-glycero-3-phosphothioethanol, and combinations thereof. In some cases, a shell (*e.g.*, a bilayer) of a structure includes 50-200 natural or synthetic lipids or lipid analogs (*e.g.*, phospholipids). For example, without limitation the shell may include less than about 500, less than about 400, less than about 300, less than about 200, or less than about 100 natural or synthetic lipids or lipid analogs (*e.g.*, phospholipids), *e.g.*, depending on the size of the structure.

Non-phosphorus containing lipids may also be used such as stearylamine, docecylamine, acetyl palmitate, and fatty acid amides. In other embodiments, other lipids such as fats, oils, waxes, cholesterol, sterols, fat-soluble vitamins (*e.g.*, vitamins A, D, E, and K), glycerides (*e.g.*, monoglycerides, diglycerides, triglycerides) can be used to form portions of a structure described herein.

A portion of a structure described herein such as a shell or a surface of a nanostructure may optionally include one or more alkyl groups, *e.g.*, an alkane-, alkene-, or alkynecontaining species, that optionally imparts hydrophobicity to the structure. An "alkyl" group refers to a saturated aliphatic group, including a straight-chain alkyl group, branched-chain alkyl group, cycloalkyl (alicyclic) group, alkyl substituted cycloalkyl group, and cycloalkyl substituted alkyl group. The alkyl group may have various carbon numbers, *e.g.*, between C₂ and C₄₀, and in some embodiments may be greater than C₅, C₁₀, C₁₅, C₂₀, C₂₅, C₃₀, or C₃₅. In some embodiments, a straight chain or branched chain alkyl may have 30 or fewer carbon atoms in its backbone, and, in some cases, 20 or fewer. In some embodiments, a straight chain or branched chain alkyl may have 12 or fewer carbon atoms in its backbone (*e.g.*, C₁-C₁₂ for straight chain, C₃-C₁₂ for branched chain), 6 or fewer, or 4 or fewer. Likewise, cycloalkyls may have from 3-10 carbon atoms in their ring structure, or 5, 6, or 7 carbons in the ring structure. Examples of alkyl groups include, but are not limited to, methyl, ethyl, propyl, isopropyl, cyclopropyl, butyl, isobutyl, tert-butyl, cyclobutyl, hexyl, cyclohexyl, and the like.

In some embodiments, the HDL-NP of the instant disclosure further comprise apolipoprotein. The apolipoprotein can be apolipoprotein A (*e.g.*, apo A-I, apo A-II, apo A-IV, and apo A-V), apolipoprotein B (*e.g.*, apo B48 and apo B100), apolipoprotein C (*e.g.*, apo C-I, apo C-II, apo C-III, and apo C-IV), and apolipoproteins D, E, and H. Additionally or alternatively, a structure described herein may include one or more peptide analogues of an apolipoprotein, such as one described above. Of course, other proteins (*e.g.*, nonapolipoproteins) can also be included in the nanostructures described herein. In some embodiments, the apolipoprotein is apolipoprotein A-I.

The HDL-NP has an organic core scaffold. An organic core scaffold as used herein refers to non-metallic material, soft-core, having a 3-dimensional structure and charge sufficient to organize and hold a lipid layer in a stable shape. In some embodiments, the shape is spherical. A "spherical" shape or structure herein refers to a structure having a round or sphere-like structure. The structure does not need to be perfectly round or an exact sphere, but rather is an approximate sphere shape.

In some embodiments, the organic core scaffold comprises a hydrophobic small molecule-phospholipid conjugate (PL₄). The hydrophobic small molecule-phospholipid conjugate comprises any small molecule capable of being linked to a phospholipid. In some embodiments, the small molecule is *tetrakis*(4-az-idophenyl)methane.

In some embodiments, the phospholipid may be a headgroup-modified phospholipid. In some embodiments, the headgroup-modified phospholipid comprises a ring-strained alkyne, 1,2-dipalmitoyl-*sn*-glycero-3-phosphoethan-olamine-N-dibenzocyclooctyl.

In other embodiments, the organic core scaffold comprises an amphiphilic DNA-linked small molecule-phospholipid conjugate (DNA- PL₄). The DNA (or any other nucleic acid, including modified and naturally occurring nucleic acids) provides a unique link between the phospholipid and small molecule. It is advantageous to use DNA because the size of the DNA and thus the core may be easily controlled by altering the length of the DNA strand. In some embodiments the DNA is 5-50 nucleotides in length In other embodiments the DNA is 5-45, 5-40, 5-35, 5-30, 5-25, 5-20, 5-17, 5-16, 5-15, 5-14, 5-13, 5-12, 5-11, 5-10, 5-9, 5-8, 5-7, 6-45, 6-40, 6-35, 6-30, 6-25, 6-20, 6-17, 6-16, 6-15, 6-14, 6-13, 6-12, 6-11, 6-10, 6-9, 6-8, 6-7, 7-45, 7-40, 7-35, 7-30, 7-25, 7-20, 7-17, 7-16, 715, 7-14, 7-13, 7-12, 7-11, 7-10, 7-9, 7-8, 8-45, 8-40, 8-35, 8-30, 8-25, 8-20, 8-17, 8-16, 8-15, 8-14, 8-13, 8-12, 8-11, 8-10, 8-9, 9-45, 9-40, 9-35, 9-30, 9-25, 9-20, 9-17, 9-16, 9-15, 9-14, 9-13, 9-12, 9-11, or 9-10 nucleotides in length.

In some embodiments, the DNA is a double stranded oligonucleotide. In some embodiments, the DNA is a double stranded oligonucleotide of 8-15 nucleotides in length. In some embodiments, the DNA is a double stranded oligonucleotide of 9 nucleotides in length.

In some embodiments, a first single strand of the double stranded DNA is linked to a phospholipid and forms a ssDNA-phospholipid conjugate (ssDNA-PL). In some embodiments, a second strand of the double stranded DNA, complementary to the first strand of the double stranded DNA is linked to a small molecule. In some embodiments, the small molecule is a tetrahedral small molecule and the small molecule linked to the DNA forms a tetrahedral small molecule-DNA hybrid (SMDH₄). In some embodiments, the SMDH₄ is linked to the ssDNA-PL through hydrogen bonding between the complementary single strands of DNA.

The small molecule may be linked directly to the phospholipid or may be linked through the use of a functional group. The functional group may include any suitable end group that can be used to functionalize the phospholipid to the small molecule, *e.g.*, an amino group (*e.g.*, an unsubstituted or substituted amine), an amide group, an azide, an imine group, a carboxyl group, or a sulfate group. In some instances, the functional group includes at least a second end group. In other embodiments, the second end group may be a reactive group that can covalently attach to another functional group. In some embodiments, the phospholipid is coupled to the small molecule with a plurality of terminal functional groups. In some embodiments, the plurality of functional groups is 2-6 functional groups. In some embodiments, the plurality of functional groups is 4 functional groups. In some embodiments, the functional groups are terminal azides (SM-Az). In an aspect, the disclosure relates to an organic core scaffold comprising, 1,2-dipalmitoyl-sn-glycero-3-phosphoethanolamine-N-dibenzocyclooctyl (DBCO PE) linked to a tetrahedral small molecule core (*tetrakis*(4-azidophenyl)methane) with 2-6 terminal azides. In some embodiments the structure has 4 terminal azides.

The size, physical properties and functional properties of the HDL-NP of the instant disclosure are similar to that of naturally occurring HDL-NP and distinct from other synthetic HDL-NP. These properties include, for example, spherical shape, surface chemistry, size, hydrodynamic diameter, zeta potential, cholesterol efflux, cholesterol delivery, and therapeutic functions such as suppression of inflammation.

In some embodiments, the HDL-NP of the instant disclosure, can be assessed based on the hydrodynamic diameter. The hydrodynamic diameter of the HDL-NP is similar to that of naturally occurring HDL-NP and distinct from other synthetic HDL-NP. Hydrodynamic diameter assesses the size of a hypothetical hard sphere that diffuses in the same manner as that of the particle being measured and provides an indication of the diffusional properties of the particle that will be indicative of the apparent size of the dynamic hydrated/solvated particle. It may be measured by Dynamic Light Scattering (DLS). In some embodiments the hydrodynamic diameter is greater than 8.7 nm. In some embodiments, the hydrodynamic diameter is 8.7 nm-17.7nm. In some embodiments, the hydrodynamic diameter is 10 nm-15nm. In some embodiments, the hydrodynamic diameter is 12 nm-14nm.

The HDL-NPs of the present disclosure can have a diameter with a largest cross-sectional dimension (or, sometimes, a smallest cross-section dimension) of, for example, less than or equal to about 500 nm, less than or equal to about 250 nm, less than or equal to about 100 nm, less than or equal to about 75 nm, less than or equal to about 50 nm, less than or equal to about 40 nm, less than or equal to about 35 nm, less than or equal to about 30 nm, less than or equal to about 25 nm, less than or equal to about 20 nm, less than or equal to about 15 nm, or less than or equal to about 5 nm. In some embodiments the HDL-NP has a diameter of about 5-30 nm, 5-25 nm, 5-22 nm, 5-20 nm, 5-15 nm, 5-14 nm, 5-13 nm, 5-12 nm, 5-11 nm, 5-10 nm, 8-15 nm, 8-14 nm, 8-13 nm, 8-12 nm, 8-11 nm, 8-10 nm, 10-12 nm, or 10 nm.

The HDL-NPs of the present disclosure can have a core with a largest cross-sectional dimension (or, sometimes, a smallest cross-section dimension) of, for example, less than or equal to about 300 nm, less than or equal to about 250 nm, less than or equal to about 100 nm, less than or equal to about 75 nm, less than or equal to about 50 nm, less than or equal to about 40 nm, less than or equal to about 35 nm, less than or equal to about 30 nm, less than or equal to about 25 nm, less than or equal to about 20 nm, less than or equal to about 15 nm, or less than or equal to about 5 nm. In some cases, the core has an aspect ratio of greater than about 1:1, greater than 3:1, or greater than 5:1. As used herein, "aspect ratio" refers to the ratio of a length or a width, where length and width are measured perpendicular to one another, and the length refers to the longest linearly measured dimension.

In some embodiments, the shell has a zeta potential closer to human HDL than a synthetic HDL nanoparticle with an inorganic core. In some embodiments, the HDL-NP has a zeta potential closer to human HDL than a synthetic HDL nanoparticle with a gold core. In some embodiments, the HDL-NP has a zeta potential of -16-26 mV. In some embodiments, the zeta potential of the HDL-like nanoparticles is about -20 millivolts (mV). In some embodiments, the zeta potential of the HDL-like nanoparticles is selected from a group consisting of -10 mV, -12 mV, -14 mV, -16 mV, -18 mV, -20 mV, -22 mV, -24 mV, -26 mV, and -30 mV. In some embodiments, the zeta potential of the HDL-like nanoparticles is greater than -20 mV. In some embodiments, the zeta potential is less than -20 mV. In some embodiments, the zeta potential is that of human HDL. Zeta potential may be assessed using methods known in the art, including the methods disclosed herein.

In some embodiments, the HDL-like nanoparticles of the present disclosure do not include a peptide-based scaffold material.

In some embodiments, the HDL-NP comprises a hydrophobic therapeutic agent. The hydrophobicity of a therapeutic agent may be assessed or measured in any way, or by any method known in the art. For example, without limitation, hydrophobicity may be measured by a partitioning method, an accessible surface area method, a chromatographic method, a physical properties method, the Wimley-White method, the Bandyopadhyay-Mehler method, and/or a combination thereof. Further, hydrophobicity of a hydrophobic therapeutic agent may also be assessed by measuring the surface polarity of the therapeutic agent. Regardless of the method used, a hydrophobic therapeutic agent will exhibit a property of being repelled and/or excluded by water. In some the hydrophobicity of the HDL-NP is measured by using a partitioning method and establishing a partition coefficient (P). In some embodiments, the solvent used in the partitioning method are water and chloroform. In some embodiments, the hydrophobic therapeutic agent has a partition coefficient (P) of greater than or equal to 0 (*e.g.*, log P ≥0). In some embodiments, the hydrophobic therapeutic agent has a partition coefficient (P) of greater than or equal to 0.2 (*e.g.*, log P ≥0.2). In some embodiments, the hydrophobic therapeutic agent has a partition coefficient (P) of greater than or equal to 0.3 (*e.g.*, log P ≥0.3). In some embodiments, the hydrophobic therapeutic agent has a partition coefficient (P) of greater than or equal to 0.5 (*e.g.*, log P ≥0.5). In some embodiments, the hydrophobic therapeutic agent has a partition coefficient (P) of greater than or equal to 0.75 (*e.g.*, log P ≥0.75). In some embodiments, the hydrophobic therapeutic agent has a partition coefficient (P) of greater than or equal to 1 (*e.g.*, log P ≥1). In some embodiments, the hydrophobic therapeutic agent has a partition coefficient (P) of greater than or equal to 2 (*e.g.*, log P ≥2). In some embodiments, the hydrophobic therapeutic agent has a partition coefficient (P) of greater than or equal to 5 (*e.g.*, log P ≥5). In some embodiments, the hydrophobic therapeutic agent has a partition coefficient (P) of greater than or equal to 6 (*e.g.*, log P ≥6). In some embodiments, the hydrophobic therapeutic agent has a partition coefficient (P) of greater than or equal to 7 (*e.g.*, log P ≥7). In some embodiments, the hydrophobic therapeutic agent has a partition coefficient (P) of greater than or equal to 10 (*e.g.*, log P ≥10).

In some embodiments, the hydrophobic therapeutic agent has a partition coefficient (P) of less than or equal to 30 (*e.g.*, log P ≤30). In some embodiments, the hydrophobic therapeutic agent has a partition coefficient (P) of less than or equal to 20 (*e.g.*, log P ≤20). In some embodiments, the hydrophobic therapeutic agent has a partition coefficient (P) of less than or equal to 15 (*e.g.*, log P ≤15). In some embodiments, the hydrophobic therapeutic agent has a partition coefficient (P) of less than or equal to 10 (*e.g.*, log P ≤10). In some embodiments, the hydrophobic therapeutic agent has a partition coefficient (P) of less than or equal to 9 (*e.g.*, log P ≤9). In some embodiments, the hydrophobic therapeutic agent has a partition coefficient (P) of less than or equal to 8 (*e.g.*, log P ≤8). In some embodiments, the hydrophobic therapeutic agent has a partition coefficient (P) of less than or equal to 7 (*e.g.*, log P ≤7). In some embodiments, the hydrophobic therapeutic agent has a partition coefficient (P) of less than or equal to 6 (*e.g.*, log P ≤6). In some embodiments, the hydrophobic therapeutic agent has a partition coefficient (P) of less than or equal to 5 (*e.g.*, log P ≤5). In some embodiments, the hydrophobic therapeutic agent has a partition coefficient (P) of less than or equal to 3 (*e.g.*, log P ≤3).

In some embodiments, the hydrophobic therapeutic agent has a partition coefficient (P) of greater than or equal to 0.2 (*e.g.*, log P ≥0.2) to less than or equal to 30. In some embodiments, the hydrophobic therapeutic agent has a partition coefficient (P) of greater than or equal to 0.3 (*e.g.*, log P ≥0.3) to less than or equal to 10 (*e.g.*, log P ≤10). In some embodiments, the hydrophobic therapeutic agent has a partition coefficient (P) of greater than or equal to 0.5 (*e.g.*, log P ≥0.5) to less than or equal to 10 (*e.g.*, log P ≤10). In some embodiments, the hydrophobic therapeutic agent has a partition coefficient (P) of greater than or equal to 0.75 (*e.g.*, log P ≥0.75) to less than or equal to 10 (*e.g.*, log P ≤10). In some embodiments, the hydrophobic therapeutic agent has a partition coefficient (P) of greater than or equal to 1 (*e.g.*, log P ≥1) to less than or equal to 10 (*e.g.*, log P ≤10). In some embodiments, the hydrophobic therapeutic agent has a partition coefficient (P) of greater than or equal to 2 (*e.g.*, log P ≥2) to less than or equal to 20 (*e.g.*, log P ≤20). In some embodiments, the hydrophobic therapeutic agent has a partition coefficient (P) of greater than or equal to 5 (*e.g.*, log P ≥5) to less than or equal to 20 (*e.g.*, log P ≤20). In some embodiments, the hydrophobic therapeutic agent has a partition coefficient (P) of greater than or equal to 6 (*e.g.*, log P ≥6) to less than or equal to 30 (*e.g.*, log P ≤30). In some embodiments, the hydrophobic therapeutic agent has a partition coefficient (P) of greater than or equal to 7 (*e.g.*, log P ≥7) to less than or equal to 30 (*e.g.*, log P ≤30). In some embodiments, the hydrophobic therapeutic agent has a partition coefficient (P) of greater than or equal to 10 (*e.g.*, log P ≥10) to less than or equal to 30 (*e.g.*, log P ≤30).

In some embodiments, the core may be tuned to the hydrophobicity of the hydrophobic therapeutic agent to be used. For example, without limitation, nucleic acids may be incorporated into the core to modulate the hydrophobicity. In some embodiments, the hydrophobicity of the core is modulated to match the hydrophobicity of the hydrophobic therapeutic agent (e.g., PIK75 (F7)). In some embodiments, the hydrophobicity of the core is modulated to be about the same hydrophobicity as the hydrophobic therapeutic agent (e.g., PIK75 (F7)). In some embodiments, the hydrophobicity of the core is modulated to be less than the hydrophobicity of the hydrophobic therapeutic agent (e.g., PIK75 (F7)). In some embodiments, the hydrophobicity of the core is modulated to be greater than the hydrophobicity of the hydrophobic therapeutic agent (e.g., PIK75 (F7)). In some embodiments, the hydrophobicity of the core is modulated to be between about 50% less hydrophobic to about 50% more hydrophobic than the hydrophobicity of the hydrophobic therapeutic agent (*e.g.*, PIK75 (F7)). In some embodiments, the hydrophobicity of the core is modulated to be between about 40% less hydrophobic to about 40% more hydrophobic than the hydrophobicity of the hydrophobic therapeutic agent (*e.g.*, PIK75 (F7)). In some embodiments, the hydrophobicity of the core is modulated to be between about 30% less hydrophobic to about 30% more hydrophobic than the hydrophobicity of the hydrophobic therapeutic agent (*e.g.*, PIK75 (F7)). In some embodiments, the hydrophobicity of the core is modulated to be between about 25% less hydrophobic to about 25% more hydrophobic than the hydrophobicity of the hydrophobic therapeutic agent (*e.g.*, PIK75 (F7)). In some embodiments, the hydrophobicity of the core is modulated to be between about 20% less hydrophobic to about 20% more hydrophobic than the hydrophobicity of the hydrophobic therapeutic agent (*e.g.*, PIK75 (F7)). In some embodiments, the hydrophobicity of the core is modulated to be between about 15% less hydrophobic to about 15% more hydrophobic than the hydrophobicity of the hydrophobic therapeutic agent (*e.g.*, PIK75 (F7)). In some embodiments, the hydrophobicity of the core is modulated to be between about 10% less hydrophobic to about 10% more hydrophobic than the hydrophobicity of the hydrophobic therapeutic agent (*e.g.*, PIK75 (F7)). In some embodiments, the hydrophobicity of the core is modulated to be between about 5% less hydrophobic to about 5% more hydrophobic than the hydrophobicity of the hydrophobic therapeutic agent (*e.g.*, PIK75 (F7)). In some embodiments, the hydrophobicity of the core is modulated to be between about 4% less hydrophobic to about 4% more hydrophobic than the hydrophobicity of the hydrophobic therapeutic agent (*e.g.*, PIK75 (F7)). In some embodiments, the hydrophobicity of the core is modulated to be between about 3% less hydrophobic to about 3% more hydrophobic than the hydrophobicity of the hydrophobic therapeutic agent (*e.g.*, PIK75 (F7)). In some embodiments, the hydrophobicity of the core is modulated to be between about 2% less hydrophobic to about 2% more hydrophobic than the hydrophobicity of the hydrophobic therapeutic agent (*e.g.*, PIK75 (F7)). In some embodiments, the hydrophobicity of the core is modulated to be between about 1% less hydrophobic to about 1% more hydrophobic than the hydrophobicity of the hydrophobic therapeutic agent (*e.g.*, PIK75 (F7)).

In some embodiments, the hydrophobic therapeutic agent is an anti-cancer agent. In some embodiments, the hydrophobic therapeutic agent is a chemotherapeutic agent. In some embodiments, the hydrophobic therapeutic agent comprises PIK75 (F7) (C₁₆H₁₄BrN₅O₄S.HCl), doxorubicin, vincristine, gemcitabine, paclitaxel, docetaxel, andrographolide, sutent, tamoxifen, or a combination thereof. In some embodiments, the hydrophobic therapeutic agent is PIK75 (F7) (C16H14BrN5O4S.HCl). In some embodiments, the hydrophobic therapeutic agent has a structure of Formula (I) (CAS No. 372196-77-5). Formula (I):

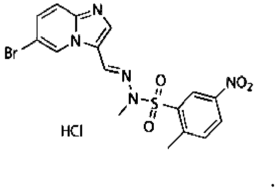

In some embodiments, the HDL-NP further comprises at least one additional therapeutic agent linked to the HDL-NP. In some embodiments, the additional therapeutic agent is a therapeutic nucleic acid. In some embodiments, the additional therapeutic agent is an anti-cancer agent. In some embodiments, the anti-cancer agent is chemotherapeutic agent.

A therapeutic nucleic acid may include any nucleic acid such as but not limited to a polynucleotide, a DNA sequence, a DNA sequence encoding a therapeutic protein, an RNA sequence, a small interfering RNA (siRNA), mRNA, a short-hairpin RNA (shRNA), a micro RNA (miRNA), an antisense oligonucleotide, a triplex DNA, a plasmid DNA (pDNA) or any combinations thereof. In some embodiments, a therapeutic nucleic acid may be treated or chemically modified. For example, a therapeutic nucleic acid may contain inter-nucleotide linkages other than phosphodiester bonds, such as phosphorothioate, methylphosphonate, methylphosphodiester, phosphorodithioate, phosphoramidate, phosphotriester, or phosphate ester linkages, which in some embodiments may confer increased stability. Nucleic acid stability may also be increased by incorporating 3'-deoxythymidine or 2'-substituted nucleotides (substituted with, *e.g.*, an alkyl group) into the nucleic acid during synthesis or by providing the nucleic acid as phenylisourea derivatives, or by having other molecules, such as aminoacridine or poly-lysine, linked to the 3' end of the nucleic acid. Modifications of a RNA and/or a DNA may be present throughout the oligonucleotide or in selected regions of the nucleic acid, *e.g.*, the 5' and/or 3' ends, for example by methylation.

In certain embodiments, the additional anti-cancer agent is a chemotherapeutic drug such as Paclitaxel, Cisplatin, Carboplatin, Topotecan, and Doxorubicin.

Thus, the HDL-NPs, compositions thereof, and methods of the present disclosure can be used in applications including, but not limited to cancer therapy. There is great promise in the use of synthetic HDLs as a therapy. Clinical trials, prior and ongoing in the field, have demonstrated that reconstituted HDLs can be safely injected in humans, and have shown marginal clinical benefit in the setting of cardiovascular disease. However, there is a need for novel approaches to synthetic HDLs that can exert more potent effects. The HDL-like nanoparticles of the present disclosure, due to their novel elements, which include hydrophobic therapeutic agents, a soft core mimic of HDL, with the ability to target cell receptors (*e.g.*, SR-B1), represent strong candidates for substantially enhanced therapeutic effects of synthetic HDL-NPs in the clinic. Accordingly, in some aspects, the disclosure relates to a method for treating a cancer, comprising administering to a subject having a cancer at least one of the high-density lipoprotein nanoparticle (HDL-NP) or compositions of the present disclosure in an effective amount to treat the cancer.

Cancers are generally characterized by unregulated cell growth, formation of malignant tumors, and invasion to nearby parts of the body. Cancers may also spread to more distant parts of the body through the lymphatic system or bloodstream. Cancers may be a result of gene damage due to tobacco use, certain infections, radiation, lack of physical activity, obesity, and/or environmental pollutants. Cancers may also be a result of existing genetic faults within cells to cause diseases due to genetic heredity. Screenings may be used to detect cancers before any noticeable symptoms appear and treatment may be given to those who are at higher risks of developing cancers (*e.g.*, people with a family history of cancers). Examples of screening techniques for cancer include but are not limited to physical examination, blood or urine tests, medical imaging, and/or genetic testing.

Non-limiting examples of cancers include: chronic myeloid leukemia (CML), adult acute myeloid leukemia (AML), acute lymphocytic leukemia (ALL), bladder cancer, breast cancer, colon and rectal cancer, endometrial cancer, kidney or renal cell cancer, leukemia, lung cancer, melanoma, Non-Hodgkin lymphoma, pancreatic cancer, prostate cancer, ovarian cancer, stomach cancer, wasting disease, and thyroid cancer. Additional non-limiting examples of cancer include Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hanlartoma, inesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinorna, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor [nephroblastoma], lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Bone: osteogenic sarcoma (osteosarcoma), fibrosarcoma, malignant fibrous histiocytoma, chondrosarcoma, Ewing's sarcoma, soft tissue Ewing's sarcoma, soft tissue sarcoma, synovial sarcoma, malignant lymphoma (reticulum cell sarcoma), multiple myeloma, malignant giant cell tumor chordoma, desmoid-type fibromatosis, fibroblastic sarcoma, gastrointestinal stromal tumors, retroperitoneal sarcoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); gynaecological sarcoma, Kaposi's sarcoma, peripheral never sheath tumor, Gynecological: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, SertoliLeydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); Hematologic: blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; Skin: malignant melanoma, basal cell carcinoma, squamous cell carcinoma, Karposi's sarcoma, moles, dysplastic nevi, lipoma, angioma, dermatofibroma, keloids, psoriasis; and Adrenal glands: neuroblastoma. In some embodiments, the subject of any one of the methods of the disclosure has cancer. In some embodiments the cancer at least one cancer selected from renal cancer, chronic myeloid leukemia (CML), multiple myeloma (MM), adult acute myeloid leukemia (AML), acute lymphocytic leukemia (ALL), cutaneous T cell lymphoma (CTCL), melanoma, ovarian cancer, breast cancer, gastrointestinal malignancies, and/or brain tumors. In some embodiments, the cancer is renal cancer. In some embodiments, the cancer is cutaneous T cell lymphoma (CTCL). In some embodiments, the cancer is colon cancer.

In some aspects, the disclosure relates to a method of delivering a hydrophobic therapeutic agent to a cell comprising a scavenger receptor class B type I (SR-BI), the method comprising administering at least one of the HDL-NPs and/or compositions of the present disclosure to a subject. In some embodiments, the cell is a cancer cell. In some embodiments, the cancer cell expresses or overexpresses scavenger receptor class B type I (SR-BI). In some embodiments, the cancer cell may be any of the cancers listed in the present disclosure. For example, without limitation, examples of cancers that express or overexpress SR-BI include human prostate cancer, breast cancer, and renal cell carcinoma. Additional non-limiting examples of cancers and cancer cell lines that overexpress SR-BI are listed in Rajora et al. Front Pharmacol. (2016) 7:326. As described herein, the term "overexpression" or "increased expression," refers to an increased level of expression of a given gene product in a given cell, cell type or cell state, as compared to a reference cell, for example, a non-cancer cell or a cancer cell that does not overexpress SR-BI. In some embodiments the cancer cell expresses any level of SR-BI.

The HDL-NPs and/or compositions administered in an effective amount can be administered for prophylactic or therapeutic treatments. As used herein, the term "treating" or "treatment" refers to the application or administration of the HDL-NPs and/or compositions thereof, to a subject who has a disease or disorder (*e.g.*, cancer), a symptom of a disease or disorder (*e.g.*, cancer), or is at risk of a disease or disorder (*e.g.*, cancer), with the purpose to prevent, cure, heal, alleviate, relieve, alter, remedy, ameliorate, improve, or affect the disorder resulting from the disease (*e.g.*, cancer). In prophylactic applications, the HDL-NPs and/or compositions can be administered to a subject (*e.g.*, patient) with a clinically determined predisposition or increased susceptibility to development of a disorder associated a hyperproliferative diseases (*e.g.*, cancer). The HDL-NPs and/or compositions of the present disclosure can be administered to the subject (*e.g.*, patient (*e.g.*, a human)) in an amount sufficient to delay, reduce, or preferably prevent the onset of the clinical disease. In therapeutic applications, HDL-NPs and/or compositions are administered to a subject (*e.g.*, patient (*e.g.*, a human) already suffering from a hyperproliferative diseases (*e.g.*, cancer) and/or other pathological conditions associated with the condition and its complications. An amount adequate to accomplish this purpose is defined as an "effective amount," "therapeutically effective amount," or "therapeutically effective dose," and is an amount of a compound (*e.g.*, HDL-NP and/or composition) sufficient to substantially improve some symptom associated with a disease or a medical condition. A therapeutically effective amount of an agent or composition is not required to cure a disease or condition but will provide a treatment for a disease or condition such that the onset of the disease or condition is delayed, hindered, or prevented, or the disease or condition symptoms are ameliorated, or the term of the disease or condition is changed or, for example, is less severe or recovery is accelerated in an individual.

As used herein, a "subject" or a "patient" refers to any mammal (e.g., a human), for example, a mammal that may be susceptible to a disease or bodily condition such as the secondary diseases or conditions disclosed herein. Examples of subjects or patients include a human, a non-human primate, a cow, a horse, a pig, a sheep, a goat, a dog, a cat or a rodent such as a mouse, a rat, a hamster, or a guinea pig. Generally, the invention is directed toward use with humans. A subject may be a subject diagnosed with a certain disease or bodily condition or otherwise known to have a disease or bodily condition. In some embodiments, a subject may be diagnosed as, or known to be, at risk of developing a disease or bodily condition. In some embodiments, a subject may be diagnosed with, or otherwise known to have, a disease or bodily condition associated with cancer, as described herein. In certain embodiments, a subject may be selected for treatment on the basis of a known disease or bodily condition in the subject. In some embodiments, a subject may be selected for treatment on the basis of a suspected disease or bodily condition in the subject. In some embodiments, the composition may be administered to prevent the development of a disease or bodily condition. However, in some embodiments, the presence of an existing disease or bodily condition may be suspected, but not yet identified, and a composition of the invention may be administered to diagnose or prevent further development of the disease or bodily condition.

In some embodiments, the HDL-NPs of the present disclosure are in a pharmaceutical composition. These "pharmaceutical compositions" or "pharmaceutically acceptable" compositions (also referred to herein simply as "compositions" of the HDL-NPs), may comprise a therapeutically effective amount of one or more of the structures described herein (*e.g.*, HDL-NPs), formulated together with one or more pharmaceutically acceptable carriers, additives, and/or diluents. The pharmaceutical compositions described herein may be useful for treating sepsis or other related diseases. It should be understood that any suitable structures described herein can be used in such pharmaceutical compositions, including those described in connection with the figures.

The pharmaceutical compositions may be specially formulated for administration in solid or liquid form, including those adapted for the following: oral administration, for example, drenches (aqueous or non-aqueous solutions or suspensions), tablets, *e.g.*, those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream or foam; sublingually; ocularly; transdermally; or nasally, pulmonary and to other mucosal surfaces.

The phrase "pharmaceutically acceptable" is employed herein to refer to those structures, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal chelating agents, such as citric acid, ethylenediamine tetraacetic acid (EDTA), sorbitol, tartaric acid, phosphoric acid, and the like.

The structures described herein may be orally administered, parenterally administered, subcutaneously administered, and/or intravenously administered. In certain embodiments, a structure or pharmaceutical preparation is administered orally. In other embodiments, the structure or pharmaceutical preparation is administered intravenously. Alternative routes of administration include sublingual, intramuscular, and transdermal administrations.

Without further elaboration, it is believed that one skilled in the art can, based on the above description, utilize the present invention to its fullest extent. The following specific embodiments are, therefore, to be construed as merely illustrative, and not limitative of the remainder of the disclosure in any way whatsoever. All publications cited herein are incorporated by reference for the purposes or subject matter referenced herein.

### Examples

### Example 1. HDL-NPs with PL₄ Cores Deliver Cytotoxic PIK75 (F7)

Cutaneous T cell lymphoma (CTCL) is a disfiguring and aggressive cancer. For patients with advanced disease, current therapies are inadequate, and outcome is poor. Incomplete understanding of CTCL molecular regulators has limited development of effective targeted therapies. One candidate regulator is p38gamma (p38γ), a mitogenactivated protein kinase downstream from the T cell receptor that is crucial for T cell activity and growth. Gene expression of the p38γ isoform is selectively increased in CTCL cell lines and patient samples, but not healthy T cells. By molecular modeling and high throughput screening, F7 (known as PIK75), a multi-kinase inhibitor affecting the p38y ATP binding site, was identified and selected as an ideal scaffold drug with significant cytotoxic impact (IC₅₀=33nM to Hut78 cells), with Kᵢ=12nM and Kₘ=3.22uM specific to p38γ (FIGs. 2A-2C). In addition, only p38γ has been identified as essential protein for CTCL growth among the four p38 isoforms by gene knockdown experiments, which makes p38γ an ideal target for the therapeutic drug development in this particular T cell lymphoma.

Additional targeting of a patient population for F7 will be for those who have renal cancer (FIGs. 3A-3C). A database analysis was performed and a survival curve for the renal cancer was found showing a separation (p=0.000084) among summarized 877 renal cancer patients by p38γ gene expression level (high vs low). Patients who had low expression of p38γ (n=366) exhibited longer life spans than that of high expression of p38γ (FIG. 3A). All 8 renal cancer cell lines have higher p38γ protein expression level than that of normal HK2 renal cells by western blot experiments (FIG. 3B). Two renal cancer cell lines, 780 and ACHN cells, were selected and their cytotoxicity IC₅₀ with respect to PIK75/F7 was determined. It was shown that 780 and ACHN cells are very sensitive to F7/PIK75 with cytotoxicity IC₅₀ of 33nM and 17nM, respectively (FIG. 3C).

Further, it was found that prostate cancer cell lines express p38γ and that several also express SR-B1. Six prostate cancer cell lines (PC-3, Du-145, CWRR1 WT (wild-type), CWRR1 EnzR (Enzalutamide resistant), LnCap WT (wild type), and LnCap EnzR (Enzalutamide resistant)) were screened for protein expression by obtaining cell lysates from the indicated cell lines and assaying by western blot for p38γ and SR-B1 expression (FIG. 6). Actin was used as a control. All tested prostate cancer cell lines expressed p38γ; and all tested cell lines except Jurkat cells expressed SR-B1.

As described elsewhere herein, PL₄ cores were synthesized in a two-step process. PL₄ core materials were synthesized by copper-free click chemistry conjugation of 1,2-dipalmitoyl-sn-glycero-3-phosphoethanolamine-N-dibenzocyclooctyl (DBCO PE) with a tetrahedral small molecule core (*tetrakis*(4-azidophenyl)methane) with four terminal azides (FIG. 1B). In a typical reaction, the DBCO PE and tetrakis(4-azidophenyl)methane were each dissolved at 0.1 wt % in N,N-dimethylformamide (DMF, Sigma Aldrich) and mixed at a 10:1 molar ratio of DBCO PE to tetrakis(4-azidophenyl)methane in DMF. The reaction mixture was subjected to three rounds of alternating vortexing and bath sonication, and was then allowed to react at room temperature under vortex for 24 h. HPLC and electrospray ionization mass spectrometry (FIG. 1C) was then used to characterize the resulting reaction mixture. As FIG. 1C only shows a single species at the right mass for PL₄, we conclude that there is no partially coupled product (PL3, PL2, *etc.*) and use the reaction mixture for the assembly step. Alternatively, the PL₄ compound can be HPLC purified at this step to remove the excess DBCO PE prior to downstream studies.

Using the resulting core scaffolds, the assembly of HDL NPs encapsulating the hydrophobic therapeutic agent (*e.g.*, PIK75/F7) was performed and optimized. A thin film was prepared of the F7 drug and PL₄ scaffold by evaporating organic solvent. Second, phosphatidylcholine (PC) liposomes were prepared in PBS via thin film formation and sonication. The PC liposomes were then added at a molar ratio of 20:1 to the thin film of PL₄ and F7. ApoA-1 was then added at a 2:1 molar ratio to the core material (PL₄ scaffold). The resultant mixture was sonicated three times (90 seconds on, 30 seconds off), and allowed to relax on ice for 30 minutes. The HDL NPs encapsulating the hydrophobic therapeutic agent (*e.g.*, PIK75/F7) were then filtered and concentrated using 50kDa molecular weight cut off columns. The concentration of HDL NPs was determined using a BCA assay to measure ApoA-1 concentration. In some embodiments of the Example, HDL NPs loaded with F7 (F7 ocHDL NPs) comprised 16 F7 molecules per HDL NP.

The HDL-NPs encapsulating PIK75/F7 ("F7 ocHDL NP") were subsequently tested for their ability to cause cytotoxicity against several cancer cell lines. Specifically, the F7-loaded HDL NPs were tested for cytoxicity against two CTCL cell lines (HH and Hut78), a clear cell renal cell carcinoma cell line (786-O), an anaplastic large cell lymphoma (ALCL) cell line (SR-786), a breast adenocarcinoma cell line (MDA MB 231), a T-cell lymphoma cell line (Jurkat), a myeloma cell line (U266B1), and six prostate cancer cell lines (PC-3, Du-145, CWRR1 WT (wild-type), CWRR1 EnzR (Enzalutamide resistant), LnCap WT (wild type), and LnCap EnzR (Enzalutamide resistant)). The HH and HuT78 cell lines are SR-B1 positive and express p38γ. The SR-786, 786-O, and MDA MB 231 cell lines are also known to be SR-B1 positive. The Jurkat and U266B1 cell lines are known to be SR-B1 negative (*i.e.,* do not express SR-B1 at detectable levels). The PC-3 and Du-145 cell lines are SR-B1 positive, androgen receptor negative and PTEN null. The CWRR1 WT cell line is SR-B1 positive, androgen receptor positive and PTEN wild type. The CWRR1 EnzR, which is also SR-B1 positive, androgen receptor positive and PTEN wild type, is a prostate cancer cell line derived from patient with castration recurrent disease that is resistant to enzalutamide (a commonly used therapy for castration-resistant prostate cancer patients). The LnCap WT cell line is SR-B1 positive, androgen receptor positive and PTEN null. The LnCap EnzR, which is also SR-B1 positive, androgen receptor positive and PTEN null, is a prostate cancer cell line derived from a lymph node metastasis that is resistant to enzalutamide.

Cells were plated into 96-well plates and subsequently treated with (i) free F7 (positive control, without HDL NPs); (ii) empty HDL NPs (negative control); (iii) HDL NPs loaded with 100:1 F7:PL4 core; (iv) HDL NPs loaded with 300:1 F7:PL4 core; or (v) HDL NPs loaded with 1000:1 F7:PL4 core. Cells were treated with varying concentrations of F7 in order to determine the half-maximal inhibitory concentrations (IC50s) for each of the tested treatment conditions. The 786-O, MDA MB 231, PC-3, Du-145, CWRR1 WT, CWRR1 EnzR, LnCap WT, and LnCap EnzR cells were incubated for four hours between the plating and treatment steps, to provide additional time for the cells to adhere to the wells. The cells were then incubated after treatment for 72 hours before assaying viability using a colorimetric assay for assessing cell metabolic activity (the MTS assay).

In each of the tested cell lines, the HDL-NPs encapsulating PIK75/F7 retained the drug sensitivity of free F7, while also demonstrating increased cytotoxic efficacy, as shown by the IC50s determined for each experiment (FIGs. 4A-4M; Table 1). Further, the data demonstrate that HDL NPs loaded with high levels of F7 (*e.g.,* 1000:1 F7:PL4 core) were more efficacious than HDL NPs loaded with relatively lower levels of F7 (*e.g.,* 100:1 F7:PL4 core). The Empty HDL NPs (negative control) did not cause cytotoxicity of any tested cell line. Collectively, this Example demonstrates that HDL NPs of the present disclosure are effective in delivering therapeutic agents (*e.g.,* hydrophobic therapeutic agents) to cells and do not negatively impact the sensitivity of the therapeutic agent following cell delivery. Furthermore, this Example suggests that the use of HDL NPs of the present disclosure are capable of treating subjects having cancer (*e.g.*, cutaneous T cell lymphoma, breast cancer, prostate cancer, and colon cancer).

**Table 1. IC-50 values (in nM) for experimental treatments**

| Cell line | Empty HDL NP | Free F7 | HDL NPs loaded with 100:1 F7:PL4 core (100X) | HDL NPs loaded with 300:1 F7:PL4 core (300X) | HDL NPs loaded with 1000:1 F7:PL4 core (1000X) |
|---|---|---|---|---|---|
| HH | N/A | 41.27 | 12.14 | 3.945 | 1.516 |
| HuT78 | N/A | 45.62 | 20.18 | 6.332 | 1.516 |
| SR-786 | N/A | 12.60 | 18.70 | 0.7428 | Not tested |
| 786-O | N/A | 282.9 | 8.679 | Not tested | 1.393 |
| MDA MB 231 | N/A | 687.9 | 45.65 | Not tested | 5.573 |
| Jurkat | N/A | 115.5 | 43.08 | 14.76 | Not tested |
| U266B1 | N/A | 44.07 | 10.72 | 6.64 | Not tested |
| PC-3 | N/A | 28.09 | Not tested | 0.4495 | Not tested |
| Du-145 | N/A | 84.66 | Not tested | 3.045 | Not tested |
| CWRR1 WT | N/A | 15.89 | Not tested | 0.3236 | Not tested |
| CWRR1 EnzR | N/A | 24.69 | Not tested | 0.3289 | Not tested |
| LnCap WT | N/A | 52.97 | Not tested | 4.855 | Not tested |
| LnCap EnzR | N/A | 75.49 | Not tested | 11.68 | Not tested |

### Example 2 Synthesis of DNA-PL₄ Cores

### Synthesis and purification of 9- SMDH₄ and 18-SMDH₄

Small molecule-DNA hybrids (SMDHs) with 9 and 18 mer DNA arms (9-SMDH₄ (SEQ ID NO: 1) and 18-SMDH₄'s (SEQ ID NO: 2), respectively) were synthesized and purified according to a previously published procedure³ and DNA sequences used in this study are listed in the Table 2. To identify the different products that were formed in the SMDH preparation, an aliquot of the collected sample of crude SMDHs was first analyzed using an analytical RP-HPLC column (see Materials and Instrumentation section herein) and a gradient method beginning with 95:5 v/v 0.1 M TEAA (aq):MeCN (TEAA (aq) = triethylammonium acetate, aqueous solution), and increasing to 60:40 v/v 0.1 M TEAA(aq):MeCN over 35 min (at a ramp of +1 vol% MeCN/min), with a flow rate of 1 mL/min. Then, the whole sample was subjected to purification using a semi-preparative RP-HPLC column (see Materials and Instrumentation section herein) and a gradient method beginning with 95:5 v/v 0.1 M TEAA (aq):MeCN and increasing to 60:40 v/v 0.1 M TEAA(aq):MeCN over 70 min (at a ramp of +0.5 vol % MeCN/min, a slower gradient was employed here to ensure adequate separation of the peaks), with a flow rate of 3 mL/min. The identity of the collected SMDH₄ product was confirmed by MALDI-ToF MS analysis (insets in FIGs. 5A-5B) and its purity was reassessed using analytical RP-HPLC (5A-5B) with the aforementioned analytical RP-HPLC solvent program.

### Solid-phase synthesis and purification of DNA-phospholipid conjugates

Syntheses were carried out from the 3' direction using controlled pore glass (CPG) beads possessing 1 µmol of either adenine (Glen Research, dA-CPG # 20-2001-10, (1000 Å, 28 µmol/g)) or thymine (Glen Research, dT-CPG # 20-2031-10 (1000 Å, 27 µmol/g)) attached to the surface. The CPG beads were placed in a 1 µmol synthesis column and 3'-phosphoramidites (Glen Research, dA-CE phosphoramidite # 10-1000-C5, Ac-dC-CE phosphoramidite # 10-1015-C5, dmf-dG-CE phosphoramidite # 10-1029-C5, dT-CE phosphoramidite # 10-1030-C5) were then added using the standard 1 µmol protocol on an Expedite 8909 synthesizer to make the CPG-3'-ssDNA (see Table 2 for sequences). A lipid phosphoramidite was added to the 5' end of ssDNA strand and then the beads were dried with a stream of dried nitrogen gas and placed in a vial containing aqueous fresh AMA solution (1 mL of a 1:1 v/v mixture of 30 wt % aqueous ammonium hydroxide solution and 40 wt % aqueous methylamine solution). The vial was then capped and heated at 65 °C for 15 min to cleave DNA-lipid conjugates from the solid supports. The ammonia and methyl amine byproducts were then removed by passing a stream of dry nitrogen gas over the content of the vial until the characteristic ammonia smell disappears. The remaining liquid, which contains the crude DNA-lipid conjugates, was collected by pipette and the remaining beads were further extracted with ultrapure deionized water (200 µL). The extract was combined with the initial solution of crude DNA-lipid conjugates (affording a total volume of 0.4 mL at the end) and filtered through a 0.45 µm nylon syringe filter (Acrodisc^{®} 13 mm syringe filter # PN 4426T). The collected sample of crude product was subjected to purification using analytical RP-HPLC (FIGs. 5C-5D) and a gradient method beginning with 95:5 v/v 0.1 M TEAA (aq):MeCN (TEAA (aq) = triethylammonium acetate, aqueous solution), and increasing to 100% MeCN over 50 min (at a ramp of +1.9 vol% MeCN/min), with a flow rate of 1 mL/min. The identity of the collected product was confirmed by MALDI-ToF analysis (insets in 5C-5D) and its purity was verified by denaturing polyacrylamide gel electrophoresis (PAGE) (FIG. 5D).

**Table 2: List of DNA Sequences of SMDH4 DNA Arms and DNA-Lipid Congugates**

| **DNA Length** | **DNA Length DNA Sequences*** | **SEQ ID NO:** |
|---|---|---|
| 9-SMDH₄ | 3'-TCG GCT GGA-small molecule | 1 |
| 18-SMDH₄ | 3'-TTG CTG AGT ATA ATT GTT-small molecule | 2 |
| 9-DNA-lipid | 3'-TCC AGC CGA-lipid | 3 |
| 18-DNA-lipid | 3'-AAC AAT TAT ACT CAG CAA-lipid | 4 |

### Assembly of DNA-phospholipid conjugates and SMDH₄

Equimolar mixtures of the as-prepared SMDH₄ and its complementary DNA-lipid conjugate in TAMg buffer solution (40 mM Tris, 20 mM acetic acid, and 7.5 mM MgCl2; pH 7.4) were added into 0.5 mL Eppendorf tubes. The resulting solutions were then heated to 90 °C in a heating block (Thermomixer R; Eppendorf, Hauppauge, NY) and kept there for 5 min to remove all initial DNA interactions. The power to the heating block was then turned off to allow the solution to slowly cool to rt over 3 h (for a typical cooling profile of this equipment, please see figure S16 in the supplementary information for Yildirim, I.; Eryazici, I.; Nguyen, S. T.; Schatz, G. C. J. Phys. Chem. B 2014, 118, 2366-2376).

### Assembly of DNA-PL₄ HDL NPs with opportunity for loading of therapeutic agents

Using the resulting DNA-PL₄ core materials, HDL NPs can then be assembled in a similar manner to HDL NPs with ordinary PL₄ cores. A therapeutic agent can be encapsulated by adding the agent at one of two steps, either in the aqueous suspension of DNA-PL₄ core or in the preparation of liposomes. Specifically, the DNA-PL₄ cores are first prepared as suspensions in aqueous buffer with or without a therapeutic agent to be encapsulated. Second, phosphatidylcholine (PC) liposomes are prepared in PBS via thin film formation and sonication with or without the presence of a therapeutic agent to be encapsulated. The PC liposomes are then added at a molar ratio of 20:1 to the aqueous suspension of DNA-PL₄. ApoA-1 is then added at a 2:1 molar ratio to the resulting suspension of DNA-PL₄ and PC lipids. The resultant mixture is sonicated three times (90 seconds on, 30 seconds off), and allowed to relax on ice for 30 minutes. The HDL NPs with or without an encapsulated therapeutic agent are then filtered and concentrated using 50kDa molecular weight cut off columns. The concentration of HDL NPs is determined using a BCA assay to measure ApoA-1 concentration.

### Example 3. Delivery of HDL-NPs with PL₄ Cores to cells via receptor-mediated update

It was demonstrated that HDL NPs loaded with F7 can be delivered to cells via receptor (SR-B1) mediated uptake. Conversely, it was demonstrated that delivery of HDL NPs does not occur via phagocytosis.

An SR-B1 positive cell line (HH cell line) and an SR-B1 negative cell line (U266B1 cell line) were treated with (i) PBS, (ii) 25 nM HDL NPs loaded with F7 ("F7 ocHDL NPs") or (iii) free F7 (concentration of 250 nM or 2.5 µM) for 2 hrs. Following treatment, the cells were washed with fresh media and plated into 96-well plates. The cells were then incubated for 72 hours prior to viability assay (MTS assay). As shown in FIG. 7A, the free F7 treatments were unable to cause cytotoxicity in the HH or U266B1 cell lines. Similarly, the F7 ocHDL NPs was unable to cause cytotoxicity in the U266B1 cell line (SR-B1 negative). However, the F7 ocHDL NPs was able to cause cytotoxicity of over 90% of total cells in the HH cell line (SR-B1 positive). These data demonstrate that the HDL NPs are capable of delivering low concentrations of hydrophobic therapeutic agents such as F7 into SR-B1 positive cells, suggesting that the HDL NPs are delivered, in some embodiments, via SR-B1 receptor-mediated uptake.

To further demonstrate that HDL NPs are capable of delivering therapeutic cargo via receptor (SR-B1) mediated uptake and not phagocytosis of the NPs, the SR-B1 positive HH cell line was pulsed with F7 ocHDL NPs (10 nM) in the presence or absence of an SR-B1 blocking antibody (used at a 50:1 media:antibody dilution).

Cells were treated with (i) PBS; (ii) 10nM F7 ocHDL NPs; or (iii) 10nM F7 ocHDL NPs + SR-B1 blocking antibody; for 2 hrs. Following treatment, the cells were washed with fresh media and plated into 96-well plates. The cells were then incubated for 72 hours prior to viability assay (MTS assay).

As shown in FIG. 7B, inclusion of the SR-B1 blocking antibody in an F7 ocHDL NP treatment prevented meaningful cytotoxicity of the HH cells, relative to the PBS control. Meanwhile, HH cells treated with F7 ocHDL NPs (but in the absence of the SR-B1 blocking antibody) experienced more than 60% cytotoxicity. These data further demonstrate that the SR-B1 receptor functions as a receptor that can mediate cellular update of HDL NPs.

### Example 4. HDL-NPs with PL₄ Cores loaded with F7 do not demonstrate a broad toxicity profile

A cell-based toxicity screen was performed to provide an initial insight towards the general toxicity profile of HDL-NPs with PL4 Cores loaded with F7. The ability of F7 ocHDL NPs to cause cytotoxicity of a hepatocellular carcinoma cell line often used to quantify toxicity towards hepatocytes (HepG2) and a common, immortalized human monocyte cell line (THP-1) was assessed. THP-1 is known to express SR-B1.

HepG2 cells were plated into 96 well plates, allowed to adhere for 4 hrs, and subsequently treated with (i) PBS; (ii) F7 ocHDL NPs (10nM, 25nM), (iii) empty ocHDL NPs (10nM or 25nM); or (iii) free F7 (250 nM or 1 µM) for 2 hrs. Following treatment, the cells were washed with fresh media and incubated for 72 hours prior to viability assay (MTS assay).

THP-1 cells were treated with (i) PBS; (ii) F7 ocHDL NPs (10 nM); or (iii) free F7 (250nM) for 2 hrs. Following treatment, the cells were washed with fresh media and plated into 96 well plates. The cells were then incubated for 72 hours prior to viability assay (MTS assay).

Neither HepG2 or THP-1 cells treated with F7 ocHDL NPs showed meaningful cytotoxicity as a result of their treatments (FIGs. 8A-8B). For HepG2 cells, this result is in contrast to the treatment of HepG2 cells with F7 (1 µM). HepG2 cells treated with F7 demonstrated mild levels of cytotoxicity. These data demonstrate that F7 ocHDL NPs (at least at the tested concentrations) do not cause cytotoxicity in selected cell lines that have been previously used for cell-based toxicity studies.

### Other Embodiments

Embodiment 1. A high-density lipoprotein nanoparticle (HDL-NP) comprising: (a) an organic core (core); (b) a shell surrounding and attached to the core wherein the core comprises a hydrophobic phospholipid conjugated scaffold (PL₄); and (c) a hydrophobic therapeutic agent associated with one or more of the organic core or shell.

Embodiment 2. The HDL-NP of embodiment 1, wherein the HDL-NP further comprises an apolipoprotein.

Embodiment 3. The HDL-NP of embodiment 2, wherein the apolipoprotein is apolipoprotein A-I (Apo-I).

Embodiment 4. The HDL-NP of: (a) embodiment 1, wherein the hydrophobic therapeutic agent is associated to the organic core and/or shell non-covalently or through hydrophobic interactions; or (b) any one of embodiments 2-3, wherein the hydrophobic therapeutic agent is associated to the organic core, shell, or apolipoprotein non-covalently or through hydrophobic interactions.

Embodiment 5. The HDL-NP of any one of embodiments 1-4, wherein the shell is attached to the organic core non-covalently.

Embodiment 6. The HDL-NP of any one of embodiments 1-5, wherein the shell is attached to the organic core through hydrophobic interactions.

Embodiment 7. The HDL-NP of embodiment 5, wherein the lipid shell is a lipid monolayer or a lipid bilayer.

Embodiment 8. The HDL-NP of any one of embodiments 1-7, wherein the PL₄ comprises a headgroup-modified phospholipid.

Embodiment 9. The HDL-NP of embodiment 8, wherein the headgroup-modified phospholipid comprises a ring-strained alkyne, 1,2-dipalmitoyl-*sn*-glycero-3-phosphoethan-olamine-N-dibenzocyclooctyl.

Embodiment 10. The spherical HDL-NP of any one of embodiments 1-9, wherein the organic core scaffold comprises an amphiphilic DNA-linked small molecule-phospholipid conjugate (DNA- PL₄).

Embodiment 11. The HDL-NP of any one of embodiments 1-10, wherein the HDL-NP has a diameter of about 5-30 nm, 5-20 nm, 5-15 nm, 5-10 nm, 8-13 nm, 8-12 nm, or 10 nm.

Embodiment 12. The HDL-NP of any one of embodiments 1-11, wherein the HDL-NP has a zeta potential closer to human HDL than a synthetic HDL nanoparticle with a gold core.

Embodiment 13. The HDL-NP of any one of embodiments 1-12, wherein the HDL-NP has a hydrodynamic diameter of 8.7 nm-17-7nm.

Embodiment 14. The HDL-NP of any one of embodiments 1-13, wherein the HDL-NP has a hydrodynamic diameter of 12 nm-14nm.

Embodiment 15. The HDL-NP of any one of embodiments 1-14, wherein the hydrophobic therapeutic agent has a partition coefficient (P) of greater than or equal to 0 (*e.g.*, log P ≥0).

Embodiment 16. The HDL-NP of any one of embodiments 1-15, wherein the hydrophobic therapeutic agent has a partition coefficient (P) of greater than or equal to 0.25 (*e.g.*, log P ≥0.25).

Embodiment 17. The HDL-NP of any one of embodiments 1-16, wherein the hydrophobic therapeutic agent has a partition coefficient (P) of greater than or equal to 0.5 (*e.g.*, log P ≥0.5).

Embodiment 18. The HDL-NP of any one of embodiments 1-17, wherein the hydrophobic therapeutic agent has a partition coefficient (P) of greater than or equal to 1 (*e.g.*, log P ≥1).

Embodiment 19. The HDL-NP of any one of embodiments 1-18, wherein the hydrophobic therapeutic agent has a partition coefficient (P) of greater than or equal to 2 (*e.g.,* log P ≥2).

Embodiment 20. The HDL-NP of any one of embodiments 1-19, wherein the hydrophobic therapeutic agent is an anti-cancer agent.

Embodiment 21. The HDL-NP of any one of embodiments 1-20, wherein the HDL-NP comprises an additional therapeutic agent.

Embodiment 22. The HDL-NP of any one of embodiments 1-21, wherein the hydrophobic therapeutic agent is a chemotherapeutic agent.

Embodiment 23. The HDL-NP of any one of embodiments 1-22, wherein the hydrophobic therapeutic agent comprises PIK75 (F7) (C₁₆H₁₄BrN₅O₄S.HCl), doxorubicin, vincristine, gemcitabine, paclitaxel, docetaxel, andrographolide, sutent, tamoxifen, or a combination thereof.

Embodiment 24. The HDL-NP of any one of embodiments 1-23, wherein the hydrophobic therapeutic agent is PIK75 (F7) (C₁₆H₁₄BrN₅O₄S.HCl).

Embodiment 25. The HDL-NP of any one of embodiments 1-24, wherein the hydrophobic therapeutic agent has a structure of Formula (I) (CAS No. 372196-77-5) 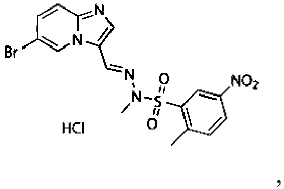 or salt thereof.

Embodiment 26. A pharmaceutical composition comprising the HDL-NP of any one of embodiments 1-25.

Embodiment 27. A method of delivering a hydrophobic therapeutic agent to a cell comprising surface receptor scavenger receptor type B1 (SR-B1) in a subject, the method comprising administering to a subject an effective amount of at least one of any one of the HDL-NPs of embodiments 1-23 and/or the composition of embodiment 24.

Embodiment 28. A method for treating a cancer, the method comprising administering to a subject having a cancer at least one of any one of the HDL-NPs of embodiments 1-23 and/or the composition of embodiment 24 in an effective amount to treat the cancer.

Embodiment 29. The method of any one of embodiments 27-28, wherein the subject is a mammal.

Embodiment 30. The method of any one of embodiments 27-29, wherein the subject is a human.

Embodiment 31. The method of any one of embodiments 27-30, wherein the subject has cancer.

Embodiment 32. The method of any one of embodiments 27-31, wherein the subject has one or more of renal cancer, chronic myeloid leukemia (CML), multiple myeloma (MM), adult acute myeloid leukemia (AML), acute lymphocytic leukemia (ALL), cutaneous T cell lymphoma (CTCL), melanoma, ovarian cancer, breast cancer, gastrointestinal malignancies, brain tumors.

Embodiment 33. The method of any one of embodiments 27-32, wherein the subject has cutaneous T cell lymphoma.

Embodiment 34. The method of any one of embodiments 27-33, wherein the subject has renal cancer.

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the claims.

### Equivalents

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.

All references, patents and patent applications disclosed herein are incorporated by reference with respect to the subject matter for which each is cited, which in some cases may encompass the entirety of the document.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, *i.e.*, elements that are conjunctively present in some cases and disjunctively present in other cases. Multiple elements listed with "and/or" should be construed in the same fashion, *i.e.*, "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, *i.e.*, the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (*i.e.* "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

### NUMBERED PARAGRAPHS

Various preferred features and embodiments of the present invention will now be described with reference to the following numbered paragraphs:
1. A high-density lipoprotein nanoparticle (HDL-NP) comprising:
   (a) an organic core;
   (b) a shell surrounding and attached to the core wherein the core comprises a hydrophobic phospholipid conjugated scaffold (PL₄); and
   (c) a hydrophobic therapeutic agent associated with one or more of the organic core or shell.
2. The HDL-NP of paragraph 1, wherein the HDL-NP further comprises an apolipoprotein.
3. The HDL-NP of paragraph 2, wherein the apolipoprotein is apolipoprotein A-I (Apo-I).
4. The HDL-NP of:
   (a) paragraph 1, wherein the hydrophobic therapeutic agent is associated to the organic core and/or shell non-covalently or through hydrophobic interactions; or
   (b) any one of paragraphs 2-3, wherein the hydrophobic therapeutic agent is associated to the organic core, shell, or apolipoprotein non-covalently or through hydrophobic interactions.
5. The HDL-NP of any one of paragraphs 1-4, wherein the shell is attached to the organic core non-covalently.
6. The HDL-NP of any one of paragraphs 1-5, wherein the shell is attached to the organic core through hydrophobic interactions.
7. The HDL-NP of any one of paragraphs 1-6, wherein the shell is a lipid shell, wherein the lipid shell is a lipid monolayer or a lipid bilayer.
8. The HDL-NP of any one of paragraphs 1-7, wherein the PL₄ comprises a headgroup-modified phospholipid.
9. The HDL-NP of paragraph 8, wherein the headgroup-modified phospholipid comprises a ring-strained alkyne, 1,2-dipalmitoyl-*sn*-glycero-3-phosphoethan-olamine-N-dibenzocyclooctyl.
10. The HDL-NP of any one of paragraphs 1-9, wherein the organic core scaffold comprises an amphiphilic DNA-linked small molecule-phospholipid conjugate (DNA- PL₄).
11. The HDL-NP of any one of paragraphs 1-10, wherein the HDL-NP has a diameter of about 5-30 nm, 5-20 nm, 5-15 nm, 5-10 nm, 8-13 nm, 8-12 nm, or 10 nm.
12. The HDL-NP of any one of paragraphs 1-11, wherein the HDL-NP has a zeta potential closer to human HDL than a synthetic HDL nanoparticle with a gold core.
13. The HDL-NP of any one of paragraphs 1-12, wherein the HDL-NP has a hydrodynamic diameter of 8.7 nm-17-7nm.
14. The HDL-NP of any one of paragraphs 1-13, wherein the HDL-NP has a hydrodynamic diameter of 12 nm-14nm.
15. The HDL-NP of any one of paragraphs 1-14, wherein the hydrophobic therapeutic agent has a partition coefficient (P) of greater than or equal to 0 (*e.g.*, log P ≥0).
16. The HDL-NP of any one of paragraphs 1-15, wherein the hydrophobic therapeutic agent has a partition coefficient (P) of greater than or equal to 0.25 (*e.g.*, log P ≥0.25).
17. The HDL-NP of any one of paragraphs 1-16, wherein the hydrophobic therapeutic agent has a partition coefficient (P) of greater than or equal to 0.5 (*e.g.*, log P ≥0.5).
18. The HDL-NP of any one of paragraphs 1-17, wherein the hydrophobic therapeutic agent has a partition coefficient (P) of greater than or equal to 1 (*e.g.*, log P ≥1).
19. The HDL-NP of any one of paragraphs 1-18, wherein the hydrophobic therapeutic agent has a partition coefficient (P) of greater than or equal to 2 (*e.g.*, log P ≥2).
20. The HDL-NP of any one of paragraphs 1-19, wherein the hydrophobic therapeutic agent is an anti-cancer agent.
21. The HDL-NP of any one of paragraphs 1-20, wherein the HDL-NP comprises an additional therapeutic agent.
22. The HDL-NP of any one of paragraphs 1-21, wherein the hydrophobic therapeutic agent is a chemotherapeutic agent.
23. The HDL-NP of any one of paragraphs 1-22, wherein the hydrophobic therapeutic agent comprises PIK75 (F7) (C₁₆H₁₄BrN₅O₄S.HCl), doxorubicin, vincristine, gemcitabine, paclitaxel, docetaxel, andrographolide, sutent, tamoxifen, or a combination thereof.
24. The HDL-NP of any one of paragraphs 1-23, wherein the hydrophobic therapeutic agent is PIK75 (F7) (C₁₆H₁₄BrN₅O₄S.HCl).
25. The HDL-NP of any one of paragraphs 1-24, wherein the hydrophobic therapeutic agent has a structure of Formula (I) (CAS No. 372196-77-5).
26. A pharmaceutical composition comprising the HDL-NP of any one of paragraphs 1-25.
27. A method of delivering a hydrophobic therapeutic agent to a cell comprising surface receptor scavenger receptor type B1 (SR-B1) in a subject, the method comprising administering to a subject an effective amount of at least one of any one of the HDL-NPs of paragraphs 1-25 and/or the composition of paragraph 26.
28. A method for treating a cancer, the method comprising administering to a subject having a cancer at least one of any one of the HDL-NPs of paragraphs 1-25 and/or the composition of paragraph 26 in an effective amount to treat the cancer.
29. The method of any one of paragraphs 27-28, wherein the subject is a mammal.
30. The method of any one of paragraphs 27-29, wherein the subject is a human.
31. The method of any one of paragraphs 27-30, wherein the subject has cancer.
32. The method of any one of paragraphs 27-31, wherein the subject has one or more of renal cancer, chronic myeloid leukemia (CML), multiple myeloma (MM), adult acute myeloid leukemia (AML), acute lymphocytic leukemia (ALL), cutaneous T cell lymphoma (CTCL), melanoma, ovarian cancer, breast cancer, gastrointestinal malignancies, brain tumors, prostate cancer, and colon cancer.
33. The method of any one of paragraphs 27-32, wherein the subject has cutaneous T cell lymphoma.
34. The method of any one of paragraphs 27-33, wherein the subject has renal cancer.
35. The method of any one of paragraphs 28-34, wherein the effective amount of the hydrophobic therapeutic agent necessary to treat the subject having cancer is decreased relative to a control (*e.g.,* a subject being treated with the same hydrophobic therapeutic agent delivered in the absence of an HDL-NP).
36. The method of paragraph 35, wherein the effective amount of the hydrophobic therapeutic agent necessary to treat the subject having cancer is decreased by at least 5%, 10%, 25%, 40%, 50%, 75%, or more, relative to a control (*e.g.,* a subject being treated with the same hydrophobic therapeutic agent delivered in the absence of an HDL-NP).
37. The method of any one of paragraphs 28-34, wherein the HDL-NPs and/or the composition cause reduced cytotoxicity of non-cancerous cells in the subject and/or reduced symptoms, relative to a control (*e.g.,* a subject being treated with a standard-of-care or alternative treatment).
38. The method of any one of paragraphs 27 or 29-34, wherein the cell is a cancer cell.

## Claims

1. A high-density lipoprotein nanoparticle (HDL-NP) comprising:
(a) an organic core, wherein the core comprises a hydrophobic phospholipid conjugated scaffold (PL₄);
(b) a shell surrounding and attached to the core; and
(c) a hydrophobic therapeutic agent associated with one or more of the organic core or shell.

2. The HDL-NP of claim 1, wherein the HDL-NP further comprises an apolipoprotein, preferably wherein the apolipoprotein is apolipoprotein A-I (Apo-I).

3. The HDL-NP of:
(a) claim 1, wherein the hydrophobic therapeutic agent is associated to the organic core and/or shell non-covalently or through hydrophobic interactions; or
(b) claim 2, wherein the hydrophobic therapeutic agent is associated to the organic core, shell, or apolipoprotein non-covalently or through hydrophobic interactions.

4. The HDL-NP of any one of claims 1-3, wherein the shell is a lipid shell, wherein the lipid shell is a lipid monolayer or a lipid bilayer.

5. The HDL-NP of any one of claims 1-4, wherein the PL₄ comprises a headgroup-modified phospholipid, preferably wherein the headgroup-modified phospholipid comprises a ring-strained alkyne, 1,2-dipalmitoyl-sn-glycero-3-phosphoethan-olamine-N-dibenzocyclooctyl.

6. The HDL-NP of any one of claims 1-5, wherein the organic core scaffold comprises an amphiphilic DNA-linked small molecule-phospholipid conjugate (DNA- PL₄).

7. The HDL-NP of any one of claims 1-6,
(a) wherein the HDL-NP has a diameter of about 5-30 nm, 5-20 nm, 5-15 nm, 5-10 nm, 8-13 nm, 8-12 nm, or 10 nm; or
(b) wherein the HDL-NP has a zeta potential closer to human HDL than a synthetic HDL nanoparticle with a gold core; or
(c) wherein the HDL-NP has a hydrodynamic diameter of 8.7 nm-17.7nm, preferably of 12nm-14nm.

8. The HDL-NP of any one of claims 1-7, wherein the hydrophobic therapeutic agent has a partition coefficient (P) of greater than or equal to 0 (*e.g.*, log P ≥0), or greater than or equal to 0.25 (*e.g.*, log P ≥0.25), or greater than or equal to 0.5 (*e.g.*, log P ≥0.5), or greater than or equal to 1 (*e.g.*, log P ≥1), or greater than or equal to 2 (*e.g.*, log P ≥2).

9. The HDL-NP of any one of claims 1-8, wherein the hydrophobic therapeutic agent is an anti-cancer agent, preferably wherein the hydrophobic therapeutic agent comprises PIK75 (F7) (C₁₆H₁₄BrN₅O₄S.HCl), doxorubicin, vincristine, gemcitabine, paclitaxel, docetaxel, andrographolide, sutent, tamoxifen, or a combination thereof.

10. The HDL-NP of any one of claims 1-9, wherein the hydrophobic therapeutic agent has a structure of Formula (I) (CAS No. 372196-77-5).

11. A pharmaceutical composition comprising the HDL-NP of any one of claims 1-10.

12. The HDL-NP of any one of claims 1-10 or the pharmaceutical composition of claim 11 for use in delivering a hydrophobic therapeutic agent to a cell comprising surface receptor scavenger receptor type B1 (SR-B1) in a subject.

13. The pharmaceutical composition of claim 11 for use in treating a cancer, in a subject in need thereof, preferably wherein the subject is a human, and preferably wherein the subject has one or more of renal cancer, chronic myeloid leukemia (CML), multiple myeloma (MM), adult acute myeloid leukemia (AML), acute lymphocytic leukemia (ALL), cutaneous T cell lymphoma (CTCL), melanoma, ovarian cancer, breast cancer, gastrointestinal malignancies, brain tumors, prostate cancer, and colon cancer.

14. The pharmaceutical composition for use according to claim 13, wherein the effective amount of the hydrophobic therapeutic agent necessary to treat the subject having cancer is decreased relative to a control subject being treated with the same hydrophobic therapeutic agent delivered in the absence of an HDL-NP.

15. The pharmaceutical composition for use according to claim 13, wherein the HDL-NPs and/or the composition causes reduced cytotoxicity of non-cancerous cells in the subject and/or reduced symptoms, relative to a standard-of-care treatment.
